(19) **Europäisches Patentamt**
**European Patent Office**
**Office européen des brevets**

(11) **EP 4 557 295 A1**

(12) **EUROPEAN PATENT APPLICATION**

(43) Date of publication:
**21.05.2025 Bulletin 2025/21**

(21) Application number: **23210835.7**

(22) Date of filing: **20.11.2023**

(51) International Patent Classification (IPC):
**G16B 15/30** $^{(2019.01)}$     **G16C 20/50** $^{(2019.01)}$

(52) Cooperative Patent Classification (CPC):
**G16B 15/30; G16C 20/50**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Dompé farmaceutici S.p.A.**
**20122 Milan (IT)**

(72) Inventors:
• **OTTAVIANI, Daniele**
**I-40033 Casalecchio di Reno (BO) (IT)**

• **MENGONI, Riccardo**
**I-40033 Casalecchio di Reno (BO) (IT)**
• **PALERMO, Gianluca**
**I-20133 MILANO (IT)**
• **TRIUZZI, Emanuele**
**I-20133 MILANO (IT)**
• **BECCARI, Andrea Rosario**
**I-20122 MILANO (IT)**
• **BONANNI, Domenico**
**I-20122 MILANO (IT)**

(74) Representative: **PGA S.p.A.**
**Via Mascheroni, 31**
**20145 Milano (IT)**

(54) **COMPUTER IMPLEMENTED METHOD FOR DETERMINING AN OPTIMAL POSITIONAL MATCHING OF A BINDING MOLECULE IN A DOCKING SITE OF A TARGET MOLECULE**

(57) It is described a computer implemented method (50) for determining an optimal positional matching of a binding molecule in a docking site of a target molecule, for example for determining an optimal positional matching of a ligand in a docking site of a target protein; the computer implemented method comprising the step (51) of obtaining a graph ($G_{mol}$) representative of the binding molecule, the step (52 of) obtaining a graph ($G_{grid}$) representative of the docking site of the target molecule, and the step (53) of correlating the graph ($G_{grid}$) representative of the docking site of the target molecule with the graph ($G_{mol}$) representative of the binding molecule for determining whether an optimal positional match exists between the binding molecule and the docking site of the target molecule. A control system (100) configure do perform the above method is also disclosed.

FIG.16

EP 4 557 295 A1

**Description**

FIELD OF THE INVENTION

The invention concerns a computer implemented method for determining an optimal positional matching of a binding molecule in a docking site of a target molecule, optionally for determining an optimal positional matching of a ligand in a docking site of a target protein.

The present invention also concerns a computer implemented method for modeling a binding molecule, for example for modeling a ligand destined to bind with a docking site of a target protein. The present invention further concerns a computer implemented method for modeling a docking site of a target molecule, for example a target protein.

The invention also concerns a computing system configured for performing the above methods. Additionally, the invention concerns a non-transitory computer-readable storage media configured to be coupled to one or more processors and having instructions stored thereon which, when executed by the one or more processors, cause the one or more processors to perform the above-mentioned methods.

The methods, systems and media of the invention may be used in the drug discovery process for facilitation identification of promising matches between a binding molecule, such as a ligand, and a target molecule, such as a protein.

BACKGROUND ART

The drug discovery process involves several tasks performed in-silico, in-vitro, and in-vivo. While the output of this process is a single valid solution, typically it starts considering a huge set of candidate molecules. In the early stages of drug discovery, the focus is on finding a small set of candidate molecules or ligands, which have a strong interaction with the binding site, named pocket, of a target molecule. These stages are performed in-silico and they usually leverage molecular docking algorithms, which estimate the strength of the interaction between the target pocket and the evaluated ligand. The strength of the interaction depends on the three-dimensional displacement of the ligand when it interacts with the target pocket. Therefore, a docking algorithm estimates the correct ligand pose to test if the ligand has to be discarded. This is a complex task due to the high number of involved degrees of freedom. Besides the six degrees of freedom for a rigid body in a three-dimensional space (three translational and three rotational), a ligand includes so called rotamers, i.e., rotational bonds between rigid fragments of the ligand). Thus, each rotamer introduces an additional degree of freedom in the problem (the angle between the two adjacent fragments).

In this situation, identifying the proper couple ligand-target molecule is a computationally complex problem.

OBJECT OF THE INVENTION

An object of the present invention is to solve the drawbacks and/or limitations of the above prior art solutions. Auxiliary objects of the invention are indicated below.

An object of the invention is to provide a new method for modelling binding molecules such as ligands and/or target molecules, such as proteins. In detail, an auxiliary object is that of identifying a modelling method allowing to implement a computationally simpler solution for determining an optimal matching of a binding molecule in a docking site of a target molecule, optionally for determining an optimal matching of a ligand in a docking site of a target protein.

A further object of the invention is to provide a new method for determining an optimal positional matching of a binding molecule in a docking site of a target molecule, optionally for determining an optimal positional matching of a ligand in a docking site of a target protein.

An additional object is implementing the above methods as computer implemented methods requiring a comparatively small computational power and yet resulting in reliable solutions.

A further object of the invention is to provide a system, and specifically a computer-system, configured for implementing one or more of the new methods.

Additionally it is a further object is that of offering a media storing instructions which when executed by a computing system configure or program the computing system to execute one or more of the above methods.

It is also an auxiliary object providing modeling methods for molecules and methods of determining the optimal matching between candidate binding molecules and docking sites of target molecules which allow the formulation of a quadratic problem particularly suitable for solution by a quantum computing system.

SUMMARY

One or more of the above objects are substantially achieved by a computer implemented method for determining an optimal positional matching of a binding molecule in a docking site of a target molecule, for example for determining an optimal positional matching of a ligand in a docking site of a target protein, as disclosed herein.

One or more of the above objects are substantially achieved by a computer implemented method for modeling a binding molecule, for example for modeling a ligand destined to bind with a docking site of a target protein, as disclosed herein.

One or more of the above objects are substantially achieved by a computer implemented method for modeling a target molecule, for example for modeling a target protein, as disclosed herein.

One or more of the above objects are furthermore achieved by a computing system configured for performing at least one the above methods.

Additionally, one or more of the delineated objects are also attained by a non-transitory computer-readable storage media carrying instructions which when processed by a computing system allow to perform at least one the above methods.

Aspects of the invention are disclosed below.

A 1<sup>st</sup> aspect concerns a computer implemented method for modeling a binding molecule, the computer implemented method comprising the steps of:

- receiving (31) a molecule description of the binding molecule to be modeled,
- representing (32) the binding molecule to be modeled as a graph ($G_{mol}$), wherein the graph ($G_{mol}$) of the binding molecule includes:

  • a plurality of nodes (1), wherein each node is representative of a respective atom of the binding molecule,
  • a plurality of first edges (2), wherein each first edge is representative of a bond between a respective pair of atoms.

In a 2<sup>nd</sup> aspect according to the 1<sup>st</sup> aspect the binding molecule object of the method for modeling is a ligand destined to bind with a docking site of a target protein.

In a 3<sup>rd</sup> aspect according to any one of the preceding aspects the computer implemented method provides that each first edge (2) has a weight representative of a length of the bond between the respective pair of atoms.

In a 4<sup>th</sup> aspect according to any one of the preceding aspects the graph ($G_{mol}$) further includes:

- one or more second edges (3), wherein each second edge is representative of a respective bond angle, wherein each bond angle is the angle between a respective pair of bonds adjacent to a same atom.

In a 5<sup>th</sup> aspect according to the preceding aspect each second edge (3) has a weight representative of an amplitude of the respective bond angle.

In a 6<sup>th</sup> aspect according to any one of the preceding aspects the graph further includes:

- one or more third edges (4), wherein each third edge is representative of a respective dihedral angle,

  wherein each dihedral angle is formed between a plane defined by a first and a second bond (A-B; B-C) and a plane defined by said second bond and a third bond (B-C, C-D),
  wherein, in a sequence of consecutively connected first, second, third and fourth atoms (A, B, C, D), the first bond (A-B) is between the first atom and the seconds atom, the second bond (B-C) is a bond between the second atom and the third atom and the third bond is a bond between the third atom and the fourth atom with the first and second bonds adjacent to the second atom (B) and with the second and third bonds adjacent to the third atom (C).

In a 7<sup>th</sup> aspect according to any one of the preceding aspects the molecule description of the binding molecule to be modeled comprises the identification of the atoms of the binding molecule.

In a 8<sup>th</sup> aspect according to any one of the preceding aspects the molecule description of the binding molecule to be modeled comprises the bonds between the binding molecule atoms.

In a 9<sup>th</sup> aspect according to any one of the preceding aspects the molecule description of the binding molecule to be modeled comprises the type of said bonds.

In a 10<sup>th</sup> aspect according to any one of the preceding aspects the step of receiving (31) a molecule description of the binding molecule to be modeled includes:

- accessing a database (103) containing a description of a plurality of molecules in a given format optionally wherein the database contains molecule descriptions in .mol2 file format;
- selecting the binding molecule to be modeled from the database; optionally selecting the binding molecule to be modeled from said database described in .mol2 file format.

In an 11<sup>th</sup> aspect according to the preceding aspect the step of receiving (31) a molecule description of the binding molecule to be modeled includes:

- accessing a database (103) containing a description of a plurality of molecules in .mol2 file format;
- selecting the binding molecule to be modeled from the database, wherein the binding molecule to be modeled selected from said database is described in .mol2 file format.

In a 12th aspect according to any one of the preceding aspects the method provides that, before or during the step of representing (32), the binding molecule as a graph ($G_{mol}$), the method comprises a step of molecule simplification (33) of the binding molecule to be modeled comprising at least removing from the binding molecule to be modeled one or more atoms.

In a 13th aspect according to the preceding aspect the step of molecule simplification (33) also comprises:

- a sub-step of reducing the number of atoms of the binding molecule to be modeled, optionally by removing the terminal hydrogens.

In a 14th aspect according to any one of the preceding two aspects the step of molecule simplification (33) also comprises:

- a sub-step of fragment removal comprising removal of one or more atoms and one or more bonds not included in a shortest path connecting adjacent rotatable bonds, and introducing one or more constraints to maintain any dihedral angle of the binding molecule to be modeled.

In a 15th aspect according to any one of the preceding three aspects the step of molecule simplification (33) also comprises:

- a sub step of fragment replacement comprising removal of one or more atoms and one or more bonds not included in a shortest path connecting adjacent rotatable bonds and replacing the removed atoms and bonds with a virtual atom optionally placed at the center of mass of the removed atoms.

A 16th aspect concerns a computer implemented method for modeling a docking site of a target molecule, the computer implemented method comprising the steps of:

- receiving (41) a description of a docking site to be modeled,
- representing (42) the docking site to be modeled as a graph, wherein the graph includes:

  • a plurality of nodes, wherein each node is representative of a respective docking point of the docking site,
  • a plurality of edges, wherein each edge is representative of a respective connection between two docking points.

In a 17th aspect according to the preceding aspect the target molecule is a protein destined to receive a ligand.

In a 18th aspect according to any one of the preceding two aspects each edge has a weight representative of the Euclidean distance between two connected docking points.

In a 19th aspect according to any one of the preceding three aspects, the description of the docking site to be modeled comprises the spatial coordinates of a plurality of docking points which are part of the docking site.

In a 20th aspect according to any one of the preceding four aspects receiving (41) a description of a docking site to be modeled comprises:

- accessing a database (103) containing a description of docking sites of a plurality of molecules in a given format;
- selecting the docking site to be modeled from the database.

In a 21st aspect according to any one of the preceding five aspects receiving (41) a description of a docking site to be modeled comprises:

- accessing a database (103) containing a description of docking sites of a plurality of molecules in a given format, wherein the database contains descriptions of docking sites of molecules in .pbp file format;
- selecting the docking site to be modeled from the database; wherein the description of the docking site to be modeled from said database is in .pbp file format.

In a 22nd aspect according to any one of the preceding six aspects the method comprises an augmentation step (43) including:

- adding one or more nodes and one or more edges to said graph representative of the docking site based on information related to a binding molecule, optionally a ligand, to be hosted in the docking site of the target molecule.

In a 23rd aspect according to any one of the preceding seven aspects the method comprises a/the augmentation step (43) including:

- based on knowledge of a distribution of the edge lengths of the graph representative of the binding molecule ($G_{mol}$), inserting nodes in the graph representative of the docking site ($G_{grid}$) that allow a similar distribution of the edge weights.

In a 24th aspect according to any one of the preceding eight aspects the method comprises a reduction step (44) including:

- removing one or more nodes and one or more edges to said graph representative of the docking site based on information related to a binding molecule, optionally a ligand, to be hosted in the docking site of the target molecule.

In a 25th aspect according to the preceding aspect the reduction step includes, based on distribution of edge lengths of the graph representative of the binding molecule ($G_{mol}$), removing from the graph representative of the docking site ($G_{grid}$) edges which lengths do not match with, or differ more than a given tolerance limit, from any length of the edges of graph representative of the binding molecule ($G_{mol}$).

In a 26th aspect according to any one of the preceding ten aspects, in combination with the 22nd aspect, the augmentation step comprises adding nodes in the graph representative of the docking site to allow in the graph representative of the docking site a distribution of the edge weights similar to that of the graph representative of the binding molecule, optionally of the ligand.

A 27th aspect concerns a computer implemented method for determining an optimal positional matching of a binding molecule in a docking site of a target molecule, for example for determining an optimal positional matching of a ligand in a docking site of a target protein, the computer implemented method comprising the steps of:

- obtaining (51) a graph ($G_{mol}$) representative of the binding molecule,
- obtaining (52) a graph ($G_{grid}$) representative of the docking site of the target molecule,
- correlating (53) the graph ($G_{grid}$) representative of the docking site of the target molecule with the graph ($G_{mol}$) representative of the binding molecule for determining whether an optimal positional match exists between the binding molecule and the docking site of the target molecule.

In a 28th aspect according to the preceding aspect the method is for determining an optimal positional matching of a ligand in a docking site of a target protein.

In a 29th aspect according to any one of the preceding two aspects the graph ($G_{mol}$) representative of the binding molecule is obtained using the method according to any one of aspects from the 1st to the 15th.

In a 30th aspect according to any one of the preceding three aspects the graph ($G_{grid}$) representative of the docking site of the target molecule is obtained using the method according to any one of aspects from the 16th to the 26th.

In a 31st aspect according to any one of the preceding four aspects determining whether an optimal positional match exists between the binding molecule and the docking site of the target molecule comprises:

- identifying one or more optimal three-dimensional poses of the binding molecule in the docking site.

In a 32nd aspect according to the preceding aspect each three-dimensional pose defines conformation, position, and orientation of the binding molecule inside the docking site.

In a 33rd aspect according to any one of the preceding six aspects the step of correlating (53) the graph ($G_{grid}$) representative of the docking site with the graph ($G_{mol}$) representative of the binding molecule comprises:

- finding (53a) whether the graph ($G_{grid}$) representative of the docking site of the target molecule contains a subgraph ($G_{grid}^{*}$) that is isomorphic to the graph ($G_{mol}$) representative of the binding molecule.

In a 34th aspect according to any one of the preceding seven aspects the step of correlating the graph ($G_{grid}$) representative of the docking site with graph ($G_{mol}$) representative of the binding molecule comprises:

- finding (53b) whether the graph ($G_{grid}$) representative of the docking site of the target molecule contains a subgraph ($G^*_{grid}$) that is a weighted isomorphism or close to a weighted isomorphism of the graph ($G_{mol}$) representative of the binding molecule.

In a 35th aspect according to the preceding aspect the step finding (53b) whether the graph ($G_{grid}$) representative of the docking site of the target molecule contains a subgraph ($G^*_{grid}$) that is a weighted isomorphism or close to a weighted isomorphism of the graph ($G_{mol}$) representative of the binding molecule comprises:

- identifying one or more optimal three-dimensional poses of the binding molecule in the docking site, by minimizing an error function correlated with the difference between the weights of the edges of the graph ($G_{mol}$) representing the binding molecule and the weights of the edges of the subgraph ($G^*_{grid}$) of the graph ($G_{grid}$) representing the docking site.

In a 36th aspect according to any one of the preceding six aspects, the step of identifying one or more optimal three-dimensional poses of the binding molecule in the docking site comprises verifying that:

$$G^*_{grid} = \left(V^*_{grid}, E^*_{grid}\right) \mid V^*_{grid} \subseteq V_{grid}, E^*_{grid} \subseteq E_{grid} \cap \left(V^*_{grid} \times V^*_{grid}\right) \qquad (1)$$

In a 37th aspect according to the preceding aspect the step of identifying one or more optimal three-dimensional poses of the binding molecule in the docking site comprises also comprises minimizing or zeroing the following error function:

$$\sum_{(u,v)\in E_{mol}} \left(w_{u,v} - w_{u',v'}\right)^2 \leq \varepsilon \; or = 0 \qquad (2)$$

with:

$$u' = f(u), u' \in V^*_{grid}$$

$$v' = f(v), v' \in V^*_{grid}$$

$$(u', v') \in E^*_{grid}$$

$$(u, v) \in \mathrm{E}_{mol}$$

meaning that for the error function to be zero (or small enough i.e. below a given threshold of acceptability $\varepsilon$) the weight associated to the edge $(u, v) \in E_{mol}$ must be equal (or almost equal) to the weight of the edge $(u', v') \in E^*_{grid}$, where $u' = f(u)$ and $v' = f(v)$, and exists (u', v') $\in$ $E^*_{grid} \subseteq E_{grid}$ for each $(u, v) \in E_{mol}$, for a given isomorphism $f$; the terms in the above expressions of the 36th and 37th aspects are as follows:

- $G_{mol} = (V_{mol}, E_{mol}, W_{mol})$ is the graph representative of the binding molecule,
- $G_{grid} = (V_{grid}, E_{grid}, W_{grid})$ is the graph representative of the docking site,
- $G^*_{grid}$ is a subgraph of $G_{grid}$ and is an isomorphism of $G_{mol}$ on $G_{grid}$,
- $V_{mol}$ are the set of nodes of $G_{mol}$,
- $E_{mol}$ are the set of edges of $G_{mol}$ ,
- $W_{mol}$ are the set of weights of the of the edges of $G_{mol}$,
- $V_{grid}$ are the set of nodes of $G_{grid}$,

- $E_{grid}$ are the set of edges of $G_{grid}$,
- $W_{grid}$ are the set of weights of the edges of $G_{grid}$,
- $V^*_{grid}$ are the set of nodes of $G^*_{grid}$,
- $E^*_{grid}$ are the set of edges of $G^*_{grid}$,
- $W^*_{grid}$ are the set of weights of the edges of $G^*_{grid}$,
- $u, v$ are the nodes of $G_{mol}$,
- $u', v'$ are the nodes of $G_{grid}$.

In a 38th aspect according to any one of the preceding eleven aspects the step of correlating (53) the graph ($G_{grid}$) representative of the docking site with the graph ($G_{mol}$) representative of the binding molecule comprises:

- in a first phase (53a) finding whether the graph ($G_{grid}$) representative of the docking site of the target molecule contains a subgraph ( $G^*_{grid}$ ) that is a non-weighted isomorphism of the graph ($G_{mol}$) representative of the binding molecule, thereby identifying pre-optimal three-dimensional poses of the binding molecule in the docking site,
- then, in a second phase (53b), selecting among the pre-optimal three-dimensional poses identified in the first phase optimal three-dimensional poses that minimize an error function correlated with the difference between the weights of the edges of the graph ($G_{mol}$) representing the binding molecule and the weights of the edges of the subgraph ( $G^*_{grid}$ ) of the graph ($G_{grid}$) representing the docking site.

In a 39th aspect according to the preceding aspect, the first phase (53a) step of identifying the pre-optimal three-dimensional poses of the binding molecule in the docking site comprises verifying that:

$$G^*_{grid} = \left(V^*_{grid}, E^*_{grid}\right) \mid V^*_{grid} \subseteq V_{grid}, E^*_{grid} \subseteq E_{grid} \cap \left(V^*_{grid} \times V^*_{grid}\right) \qquad (1)$$

In a 40th aspect according to the preceding aspect, the second phase (53b) step of selecting, among the pre-optimal three-dimensional poses identified in the first phase, optimal three-dimensional poses comprises finding the three-dimentional poses that minimize or zero the following error function:

$$\leq \mathcal{E} \ or \qquad (2)$$

$$\sum_{(u,v)\in E_{mol}}\left(w_{u,v} - w_{u',v'}\right)^2 = 0 \qquad (2)$$

(PAG 21)
with:

$$u' = f(u), u' \in V^*_{grid}$$

$$v' = f(v), v' \in V^*_{grid}$$

$$(u', v') \in E^*_{grid}$$

$$(u, v) \in E_{mol}$$

meaning that for the error function to be zero (or small enough i.e. below a given thrshold of acceptability) the weight associated to the edge $(u, v) \in E_{mol}$ must be equal (or almost equal) to the weight of the edge $(u', v') \in E^*_{grid}$,

where $u' = f(u)$ and $v' = f(v)$, and exists $(u', v') \in$ $E_{grid}^* \subseteq E_{grid}$ for each $(u, v) \in E_{mol}$, for a given isomorphism $f$; the terms in the above expressions of the 39th and 40th aspects are as follows:

- $G_{mol} = (V_{mol}, E_{mol}, W_{mol})$ is the graph representative of the binding molecule,
- $G_{grid} = (V_{grid}, E_{grid}, W_{grid})$ is the graph representative of the docking site,
- $G_{grid}^*$ is a subgraph of $G_{grid}$ and is an isomorphism of $G_{mol}$ on $G_{grid}$,
- $V_{mol}$ are the set of nodes of $G_{mol}$,
- $E_{mol}$ are the set of edges of $G_{mol}$,
- $W_{mol}$ are the set of weights of the of the edges of $G_{mol}$,
- $V_{grid}$ are the set of nodes of $G_{grid}$,
- $E_{grid}$ are the set of edges of $G_{grid}$,
- $W_{grid}$ are the set of weights of the edges of $G_{grid}$,
- $V_{grid}^*$ are the set of nodes of $G_{grid}^*$,
- $E_{grid}^*$ are the set of edges of $G_{grid}^*$,
- $W_{grid}^*$ are the set of weights of the edges of $G_{grid}^*$,
- $u$, $v$ are the nodes of $G_{mol}$,
- $u'$, $v'$ are the nodes of $G_{grid}$.

In a 41st aspect according to any one of the preceding thirteen aspects the step of correlating (53) the graph ($G_{grid}$) representative of the docking site with graph ($G_{mol}$) representative of the binding molecule comprises constructing (53c) a corresponding Quadratic Unconstrained Binary Optimization (QUBO) problem.

In a 42nd aspect according to the preceding aspect, given the graph ($G_{grid}$) representative of the docking site and the graph ($G_{mol}$) representative of the binding molecule, constructing a corresponding Quadratic Unconstrained Binary Optimization (QUBO) problem comprises:

- defining a set of binary variables:

$$x_{u,u'} = \begin{cases} 1 & \text{if vertex } u \text{ is mapped to vertex } u' \\ 0 & \text{otherwise} \end{cases}$$

$$\forall u \in V_{mol}, \quad \forall u' \in V_{grid}$$

- mapping each node of the graph of the binding molecule ($G_{mol}$) to one and only one node of the docking site graph ($G_{grid}$):

$$H_1 = \sum_u \left(1 - \sum_{u'} x_{u,u'}\right)^2, \qquad u \in V_{mol}, u' \in V_{grid}$$

- mapping each edge of the graph of the binding molecule ($G_{mol}$) to edges of the docking site graph ($G_{grid}$):

$$H_2 = \sum_{(u,v)\in E_{mol}} \sum_{(u',v')\notin E_{grid}} x_{u,u'} x_{v,v'}$$

- defining an isomormism term:

$$H_{iso} = H_1 + H_2$$

In a 43rd aspect according to the preceding aspect, constructing said corresponding Quadratic Unconstrained Binary Optimization (QUBO) problem further comprises:

- defining an optimization term:

$$H_{opt} = \sum_{(u,v)\in E_{mol}} \sum_{(u',v')\in E_{grid}} \left(w_{u,v} - w_{u',v'}\right)^2 x_{u,u'} x_{v,v'}$$

meaning that if the binary variables

$x_{u,u'}$, which map node $u \in V_{mol}$ on node $u' \in V_{grid}$, and
$x_{v,v'}$, which maps node $v \in V_{mol}$ on node $v' \in V_{grid}$

are equal to 1, then a score is evaluated equal to the squared module of the difference between the edge weights associated to the edges $(u, v) \in E_{mol}$ ($w_{u,v} \in W_{mol}$) and $(u', v') \in E_{grid}$ ($w_{u',v'} \in W_{grid}$).

In a 44th aspect according to the preceding aspect, constructing said corresponding Quadratic Unconstrained Binary Optimization (QUBO) problem comprises:

- defining a complete Hamiltonian ( $\mathcal{H}_{MD}$ ) associated to the Quadratic Unconstrained Binary Optimization (QUBO) problem formulation as:

$$\mathcal{H}_{MD} = AH_{iso} + H_{opt}$$

$$= A(H_1 + H_2) + H_{opt}$$

$$= A\left(\sum_u \left(1 - \sum_{u'} x_{u,u'}\right)^2 + \sum_{(u,v)\in E_{mol}} \sum_{(u',v')\notin E_{grid}} x_{u,u'} x_{v,v'}\right)$$

$$+ \sum_{u,v\in E_{mol}} \sum_{u',v'\in E_{grid}} \left(w_{u,v} - w_{u',v'}\right)^2 x_{u,u'} x_{v,v'}$$

with (A) being an optimization parameter.

In a 45th aspect according to the preceding aspect, optimal three-dimensional poses that minimize an error function correlated with the difference between the weights of the edges of the graph ($G_{mol}$) representing the binding molecule and the weights of the edges of the subgraph ( $G^*_{grid}$ ) of the graph ($G_{grid}$) representing the docking site are found by minimizing said complete Hamiltonian ( $\mathcal{H}_{MD}$ ).

In a 46th aspect according to one of the preceding two aspects the Quadratic Unconstrained Binary Optimization (QUBO) problem is solved and/or the optimal three-dimensional poses are determined by a Quantum Annealing (QA) optimization process executed by a quantum annealer.

In a 47th aspect according to one of the preceding three aspects the optimization parameter (A) is such that:

$$A \geq \max_{(u,v)\in E_{mol},(u',v')\in E_{grid}} \left(w_{u,v} - w_{u',v'}\right)^2$$

In a 48th aspect according to the preceding aspect, the optimization parameter (A) is:

$$A = 10 \cdot \text{max\_optimization\_coefficient}$$

$$\text{max\_optimization\_coefficient} = \max_{(u,v) \in E_{mol}, (u',v') \in E_{grid}} \left( w_{u,v} - w_{u',v'} \right)^2$$

A 49th aspect concerns a computer implemented method for identifying from a list of numerous candidate binding molecules one or more binding molecules that represent a promising match in a docking site of a target molecule, for example for identifying from a list of numerous ligands the one or more that represent promising matches with a protein pocket,

the method comprising:

- executing the method according to any one of the preceding aspects from the 27th to the 49th aspect for each candidate binding molecule,
- identifying among the numerous candidate binding molecules one or more binding molecules that represent a promising match in the docking site of the target molecule as those binding molecules for which an optimal positional matching in the docking site of the target molecule has been determined.

In a 50th aspect the one or more binding molecules that represent a promising match in the docking site of the target molecule are those binding molecules for which one or more optimal three-dimensional poses of the binding molecule in the docking site have been identified.

A 51st aspect concerns a computing system, comprising:

one or more processors; and
a computer-readable storage device coupled to the one or more processors and having instructions stored thereon which, when executed by the one or more processors, cause the one or more processors to perform the method of any one of the preceding aspects.

A 52nd aspect concerns a non-transitory computer-readable storage media couplable to one or more processors and having instructions stored thereon which, when executed by the one or more processors, cause the one or more processors to perform the method of any one of the preceding aspects from the 1st aspect to the 50th aspect.

BRIEF DESCRIPTION OF THE DRAWINGS

Some embodiments and some aspects of the invention are described hereinafter with reference to the accompanying drawings, provided only for illustrative and, therefore, non-limiting purposes, in which:

> Figures 1 and 2 are graph representations of a binding molecule;
> Figure 3 is a schematic view showing the bond angle between two edges exiting from a common node;
> Figures 4 and 5 are further graph representations of a binding molecule;
> Figure 6 is a schematic view showing the torsion angle, also known as the dihedral angle, between the A-B bonds and the D-C bonds when considering four atoms connected in the order A-B-C-D.
> Figure 7 is a schematic view of a molecule and of an associated graph representation;
> Figures 8-12 show schematic representations of a molecule in various steps of a molecule simplification phase;
> Figure 13 is a schematic view of a computing system for executing the methods described herein, according to aspects of the invention;
> Figure 14 is a block diagram of a computer implemented method used to model a binding molecule according to aspects of the invention;
> Figure 15 is a block diagram a computer implemented method is used to model a docking site of a target molecule according to aspects of the invention; and
> Figure 16 is a block diagram representative of a computer implemented method to find an optimal positional matching of a binding molecule (e.g., a ligand) in a docking site of a target molecule (e.g., a pocket of a protein), according to further aspects of the invention.

CONVENTIONS and DEFINITIONS

In the present detailed description and in the figures corresponding parts are indicated by the same reference numerals. The figures may show representations that are not in scale; furthermore, parts and components illustrated in the figures may be in the form of schematic representations.

Note that here below and in the claims, binding molecules may, for example, be ligands and target molecules may, for example, be proteins. Thus, when a method or method step is described (for example a method for modeling a molecule or a method for finding the best match or matches between a list of numerous ligands and a pocket of a target protein), this should not be interpreted in a limiting manner and it should be appreciated that the methods described and claimed herein may be applied to binding molecules different from ligands and to target molecules different from proteins.

Bond order refers to the number of chemical bonds formed between two atoms in a molecule. For example, two atoms of hydrogen combine to form a hydrogen molecule (H-H or $H_2$). Therefore, the bond order of $H_2$ is one. Similarly, the bond order of oxygen (O=O or $O_2$) is two and that of nitrogen (N≡N or $N_2$) is three. The value need not be an integer, especially for polyatomic molecules having resonance structures. The bond order indicates the stability of the bond. It is inversely proportional to the bond length. The shorter the bond length, the higher is the bond order.

The bond order can be determined from the Lewis structure using the following steps:

Step 1: Draw the Lewis structure
Step 2: Allocate the following bond order for different types of bonds:

0: No bond
1: Single covalent bond
2: Double covalent bond
3: Triple covalent bond

Step 3: Count the total number of bonded pairs of atoms or bond group
Step 4: Add the bond order of each bond group
Step 5: Divide the total bond order by the number of bond groups to get an average bond order.

Bond length describes the distance between the nuclei of bonded atoms. The length of a bond is inversely proportional to the bond order; the higher the bond order, the shorter the length of the bonds. This occurs as the higher bond order causes an increased amount of attraction between the atoms, resulting in a shortened bond. Bond length is measured in pm (1 picometer → $10^{-12}$ meters), or A°(1 Angstrom → $10^{-10}$ meters), see for example the table below showing bond length between atoms of certain molecules.

| Bond | Bond Length (pm) | Bond Length (Å) |
|---|---|---|
| C - C | 154 | 1.54 |
| C = C | 134 | 1.34 |
| C ≡ C | 120 | 1.20 |
| N - N | 147 | 1.47 |
| N = N | 124 | 1.24 |
| N ≡ N | 110 | 1.10 |

*Bond length examples in picometer and Angstrom*

Bond lengths can be considered fixed: they cannot be compressed or elongated at will. At short distances, the hard-sphere boundary prevents the interaction from becoming positive. At long distances, the spinodal condition prevents the bond force constant to be negative. Both are intrinsic consequences of the bond length at equilibrium.

DETAILED DESCRIPTION

A. General introduction

Molecular Docking (MD) is an important step of the drug discovery process which aims at calculating the preferred position and shape of a first molecule with respect to a second molecule when they are bound to each other.

The MD process is divided into two main tasks:

- Shape Complementarity (SC) search: detection of three-dimensional poses of the molecule (ligand), i.e. valid ligand conformations, positions, and orientations inside the active site of the target molecule (protein), also known as pocket.
- Binding Affinity (BA) evaluation: ranking of the poses via a scoring function. Usually the lower the docking score, the better the resulting binding affinity. The scoring functions are limited to estimate, rather than to calculate, the binding affinity between the protein and ligand. In order to do so, assumptions and simplifications are leveraged. Scoring functions can be categorized based on whether they use physical features of the ligand and of the pocket, chemical features, or statistical knowledge of the interactions between classes of chemical compounds and some typical patterns in the shape of the pockets.

The computer implemented methods, computing systems and memory media of the invention may be used in the drug discovery process, and for example in the SC search for performing one or more of the following:

- efficiently modelling the structure and geometry of a binding molecule, for example of a ligand,
- efficiently modeling the structure and geometry of a target molecule or of a pocket in a target molecule, such as a pocket of a protein,
- facilitating identification of promising matches between a binding molecule, such as a ligand, and a target molecule, such as a protein,
- solving the so called "molecular docking (MD) problem", namely determining out of a list of numerous binding molecules those that represent the promising matches with a target molecule docking site, for example determining out of a list of numerous ligands those that represent promising matches with a protein pocket.

The computer implemented methods may include the aspects summarized below.

In accordance with one aspect, it has been determined that a complex object as a molecule or a part of it, such as a docking site or pocket, may be simplified considering only structural information: the atoms, the relative bonds between the atoms and, optionally, other constraints.

In practice, a candidate binding molecule is treated as a structure that must bind with the structure of a docking site of a target molecule to perform the docking, at least from a geometrical point of view. The same approach may be applied to the target protein pocket, which can be seen as a three-dimensional volume where the binding molecule may fit with rigid roto-translation and additional torsions provided by the internal degrees of freedom of the binding molecule.

As further described below, and in accordance with a further aspect, the candidate binding molecules are properly modeled using respective graphs, the docking sites or pockets of the target molecule or molecules are also modeled using respective graphs and then the search for the most fit compound binding molecule-target molecule is made appropriately correlating the binding molecules graphs with the graph of the target molecule as further explained below.

In further aspects, the best match or matches between a list of numerous ligands (or other candidate binding molecules) and a pocket of a target protein (or other target molecule) is sought selecting the best fits among the tested candidates. In a possible aspect, the proposed approach tackles the SC search phase by formulating the problem in terms that can be more easily digested by an annealing approach. The new approach is more suited to a Quadratic Unconstrained Binary Optimization (QUBO) formulation, which is natively used for optimization problems solved, e.g., with Quantum Annealing.

In accordance with an aspect of the proposed approach, docking points within the pocket (e.g. of a protein) which identify an active region of the pocket itself are selected. The number of docking points depends on the pocket shape and dimension and are generated using methods derived from the literature (e.g., CAVIAR, PASS, POCASA). The docking points of the pocket can be seen as the vertices of a weighted spatial grid that identifies a certain discretization of the 3D space region inside the pocket, where weights represent distances between docking points.

Ligands (or other binding molecules), on the other hand, are represented via weighted graphs that embed geometrical properties of the molecule like connectivity between atoms, rotatable bonds, bond lengths, and values of fixed angles.

Finally, ligand (or other binding molecule) poses are evaluated in terms of a weighted subgraph isomorphism between the ligand graph and the space grid of the pocket. This approach has the power of being natively formulated as a QUBO problem thus avoiding the wasteful overhead in the number of resources generally associated with the transition from High Order Binary Optimization (HUBO) problem to QUBO.

The optimization on the weights enables the search for configurations in which the geometry is maintained, while the subgraph isomorphism allows the rotatable bonds to rotate in space and the ligand/binding molecule as a whole to roto-translate within the space grid of the pocket.

B. The computing system

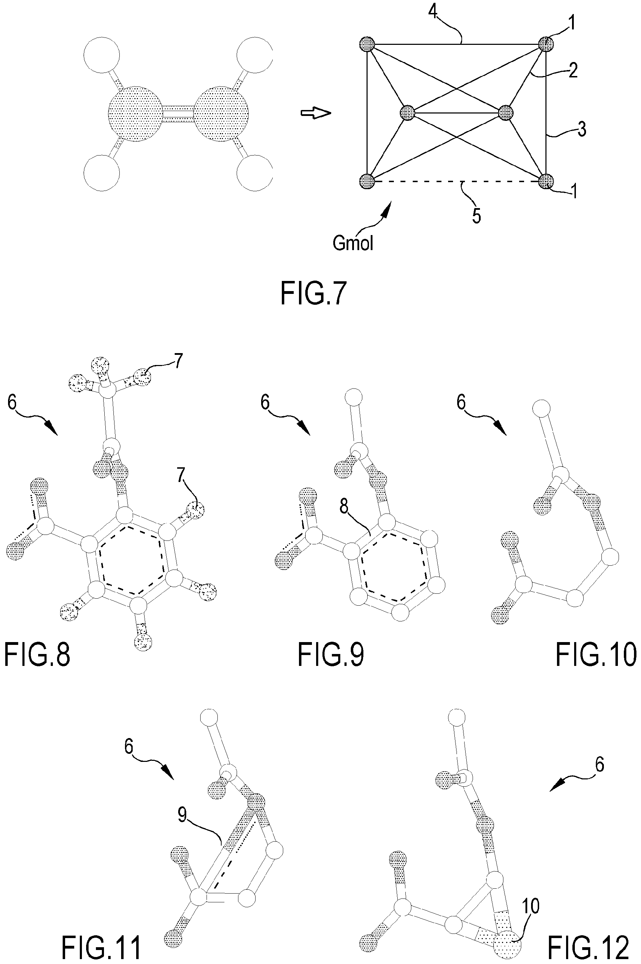

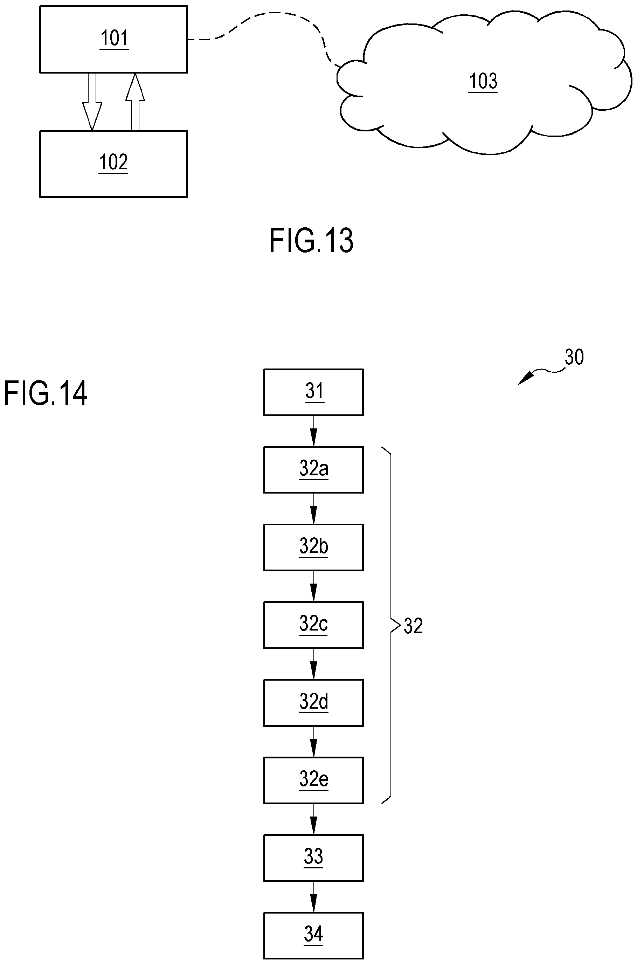

As already mentioned the methods disclosed herein are computer implemented methods. These methods may be executed by a system 100 (see figure 10), for example a computing system, comprising one or more processors 101 and a computer-readable storage device 102 coupled to the one or more processors 101 and having instructions stored thereon which, when executed by the one or more processors, cause the one or more processors to perform the methods disclosed herein and the methods of any one of the preceding claims. The computing system 100 may also be communicatively connected with one or more databases 103 for receiving information relating to molecules as further described below. The methods disclosed and claimed herein may also be executed by one or more processors such as processor(s) 101 of Fig. 13 capable of executing appropriate instructions stored in a non-transitory computer-readable storage media couplable to the one or more processors.

As used herein the term "computing system" encompasses all apparatus, devices, and machines for processing data, including by way of example a programmable processor, a computer, or multiple processors or computers. The apparatus may include, in addition to hardware, code that creates an execution environment for a computer program (e.g., code that constitutes processor firmware, a protocol stack, a database management system, an operating system, or any appropriate combination of one or more thereof). A computer program (also known as a program, software, software application, script, or code) may be written in any appropriate form of programming language, including compiled or interpreted languages, and it may be deployed in any appropriate form, including as a stand-alone program or as a module, component, subroutine, or other unit suitable for use in a computing environment. A computer program does not necessarily correspond to a file in a file system. A program may be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program may be deployed to be executed on one computer or on multiple computers that are located at one site or distributed across multiple sites and interconnected by a communication network.

The processes and logic flows described in this specification may be performed by one or more programmable processors executing one or more computer programs to perform functions by operating on input data and generating output. The processes and logic flows may also be performed by, and apparatus may also be implemented as, special purpose logic circuitry (e.g., an FPGA (field programmable gate array) or an ASIC (application specific integrated circuit), a GPU (Graphics Processing Unit), a TPU (Tensor Processing Unit), a quantum computer.

Processors suitable for the execution of a computer program include, by way of example, both general and special purpose microprocessors, and any one or more processors of any appropriate kind of digital computer. Generally, a processor will receive instructions and data from a read only memory or a random access memory or both. Elements of a computer can include a processor for performing instructions and one or more memory devices for storing instructions and data. Generally, a computer will also include, or be operatively coupled to receive data from or transfer data to, or both, one or more mass storage devices for storing data (e.g., magnetic, magneto optical disks, or optical disks). However, a computer need not have such devices. Moreover, a computer may be embedded in another device (e.g., a mobile telephone, a wearable computing device (e.g., smart watch, smart wristband, smart ring), a personal digital assistant (PDA), a mobile audio player, a Global Positioning System (GPS) receiver). Computer readable media suitable for storing computer program instructions and data include all forms of non-volatile memory, media and memory devices, including by way of example semiconductor memory devices (e.g., EPROM, EEPROM, and flash memory devices); magnetic disks (e.g., internal hard disks or removable disks); magneto optical disks; and CD ROM and DVD-ROM disks. The processor and the memory may be supplemented by, or incorporated in, special purpose logic circuitry.

C. From binding molecule (e.g., ligand) to molecule graph

In accordance with an aspect, a computer implemented method is used to model a binding molecule is now described. This method and the related method steps are schematically represented by the block diagram of Fig. 14, wherein the method is globally identified with reference numeral 30.

A description of the binding molecule may already be available to the computing system 100 or for example a description may obtained from a database 103 of molecules communicatively connected with the computing system 100 (step 31). The computing system 100 may access database 103 containing a description of a plurality of molecules in a given format (for example the database may contain molecule descriptions in .mol2 file format) and then select the binding molecule to be modeled from the database.

The computing system 100, which is able to execute the methods described herein, starting from the molecule description for example in .mol2 format, obtains a graph ($G_{mol}$) that represents the molecule geometry and that respects the constraints of bond lengths and for example also bond angles by introducing weighted edges. It is noted that, irrespective of the format used, the molecule description of the binding molecule to be modeled may comprise at least the identification of: the atoms of the binding molecule, the bonds between the binding molecule atoms, and the type of said bonds.

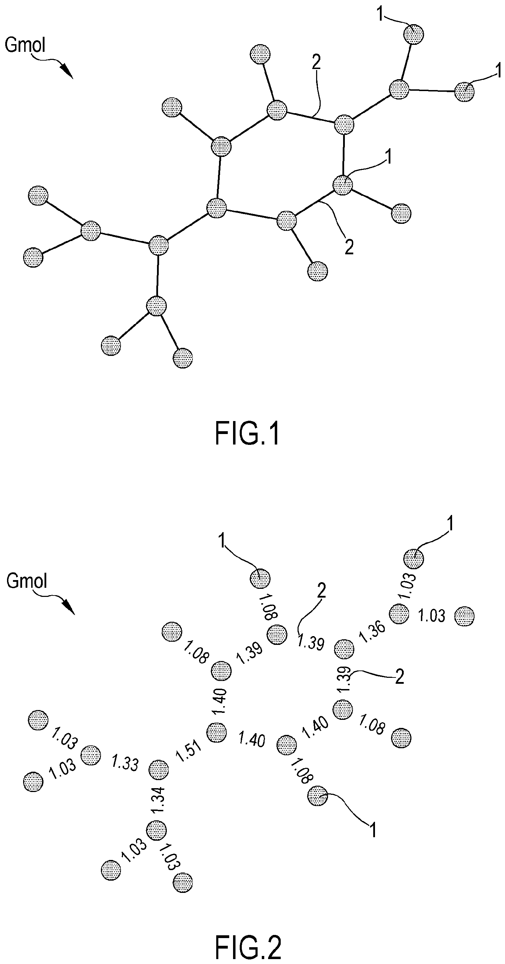

As already mentioned, the binding molecule (ligand) to be modeled is converted (step 32 in Fig. 14) into graph ($G_{mol}$), which is schematically represented in Fig. 1. The graph ($G_{mol}$) is capable of representing the molecule features and geometric constraints. The graph ($G_{mol}$) representative of the binding molecule may then be conveniently used together with a graph representative of the target molecule (protein) to determine if the two molecules in question acceptably match, as further explained herein below.

In greater detail, once obtained a molecule description e.g., in .mol2 format, to map a molecule into graph ($G_{mol}$)

(see again Fig. 1) each atom of the molecule is represented (sub-step 32a) by one of the nodes 1 (or vertexes) of the graph. Furthermore, to connect the nodes 1 of the graph in order to keep a coherent structure of the molecule, edges 2 are added between the nodes 1 associated with atoms with non-zero bond order (sub-step 32b). In other words, bonds between a respective pair of atoms of the molecule are represented by edges 2 in the graph (here below also indicated as first edges 3 in Fig. 1).

In a further aspect, in order to respect the bond lengths between the pair of atoms of the binding molecule, the edges 2 (first edges) are weighted edges. This is represented in Fig. 2: in other words the edges are enriched with a weight information (sub-step 32 c) representative of the bond length between two concerned atoms. The weight is equal to the distance between the two atoms, expressed in Angstrom or picometers.

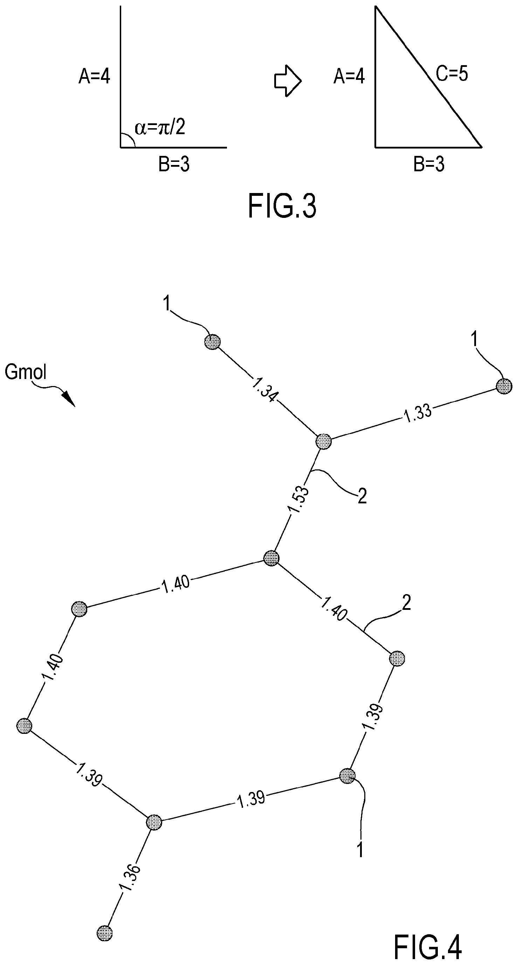

In order to provide an even better model of the binding molecule, a further fixed geometric feature that may be taken into consideration is the bond angle between a respective pair of bonds adjacent to a same atom. According to a further aspect, each bond angle may be expressed in the molecule graph as an additional edge (or second edge) 3 that has a weight representative of an amplitude of the respective bond angle (sub step 32d). In practice, as shown in the example of Fig. 3, the bond angle between two edges exiting from a common node can be identified by adding a length constraint between the two adjacent edges, i.e. between the two nodes at the end of the two adjacent edges and opposed to the common node. In the case shown in figure 3, the two adjacent edges measure respectively A=3 and B=4 of a given unit and by imposing that the 3$^{rd}$ edge shown measures C=5 (with A, B, C measured with a same unit for example picometers), the result is that the angle between A and B is 90°.

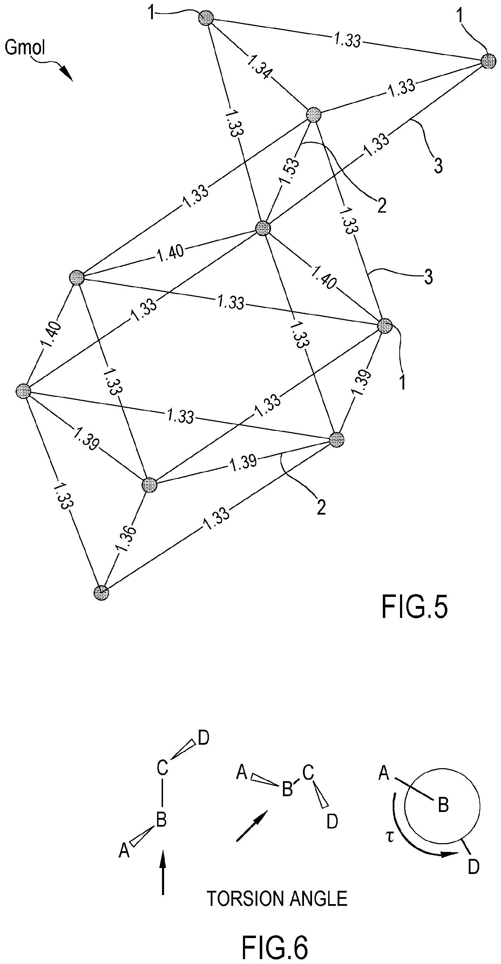

Fig. 4 and Fig. 5 show a molecule graph respectively before and after adding the second edges 3 that replace the bond angles constraint.

In conclusion, it is possible, starting from a molecule in .mol2 format, to obtain a graph that represents its geometry and that respects the constraints of bond lengths and bond angles by introducing weighted edges. Each second edge 3 has a weight representative of an amplitude of the respective bond angle.

In addition to bond lengths and bond angles, another parameter that may be taken into account in describing the conformation of molecules is the torsion angle. The torsion angle, also known as the dihedral angle, is the relative angle, between the A-B bonds and the D-C bonds when considering four atoms connected in the order A-B-C-D. The torsion or dihedral angle can also be considered as the angle between two planes defined as A-B-C and B-C-D. Rotations around a B-C single bond lead therefore to different torsion angles. The torsion angle is frequently called $\tau$ (see Fig. 6). By convention, following the definition proposed by W. Klyne and V. Prelog (Experientia, 1960, 16, 521-523, which is incorporated herein by reference), a positive value of the torsion angle A-B-C-D is assigned to the clockwise rotation of up to 180° necessary to bring the front atom into an eclipsed position with the rear atom. This being said, it is noted that molecules may have rotatable bonds. A rotatable bond is defined as any single non-ring bond, attached to a non-terminal, non-hydrogen atom. In those cases, the torsional angle should not be considered fixed. Rotatable bonds add degrees of freedom on top of the six degrees of freedom for a generic rigid body in a three-dimensional space (three translational and three rotational degrees of freedom). In particular, single bonds in organic molecules are free to rotate, due to the end-to-end nature of their orbital overlap. Double and triple bonds are not rotatable since pi bonds (created from overlapping p orbitals) prevent rotations of the dihedral angles. Since in certain molecules some dihedral angles are fixed, in accordance with a further aspect it is envisaged that the graph representative of the binding molecule or ligand may also represent this constraint. To cope with this, one or more other edges (or third edges) 4, 5 may be added to the molecule graph (step 32e in Fig. 14), between the nodes representative of atom A and atom D for each dihedral angle A-B-C-D that needs to be fixed. With this strategy, it is possible to represent topologically the non-rotation constraint between the four atoms. In Fig. 7, it is shown how to fix the dihedral angle in the graph associated with the ethane: it is enough add one of the two edges 4, 5.

Once the described constraints have been defined in graph ($G_{mol}$), the geometry of the binding molecule is represented by edges and edge weights of the same graph ($G_{mol}$). Therefore, each graph ($G_{mol}$) as above defined does not introduce distortions of the bond lengths, of the bond angles, and of the fixed dihedral angles associated to non-rotatable bonds. On the other hand, the graph representation ($G_{mol}$) does not constrain the rotations of rotatable bonds. In other words, two identical molecules which differ from a rotation of a rotatable bond give rise to the same graph: the same set of nodes, and the same set of edges (and edge weights), since the bond lengths and the bond angles are independent to the configuration of the dihedral angles associated to rotatable bonds.

In accordance with a further aspect, the computer-implemented method described herein may include a step of molecule simplification (step 33) aiming at reducing the modelled size of the molecule without substantially impacting on its geometrical behavior during docking evaluation and at the same time reducing the computational demand on computing system 100.

A first molecule simplification includes reducing the number of atoms of the molecule to be modeled for example by removing the terminal hydrogens. It is possible to see, from a comparison between the molecule before (fig. 8 shows a given starting molecule 6 with terminal hydrogens 7) and after the removal of the terminal hydrogens 6 (Fig. 9), how the molecule 6 and consequently the associated graph ($G_{mol}$) that will be determined get simpler. In other words, by removing some simple atoms, such as the terminal hydrogens, the impact in term of simplification cannot be ignored: both the number of edges and the number of nodes decrease. Therefore, also the docking task becomes simpler and the lost information does not heavily impact on the quality of the model and on reliability of the docking task.

In a further aspect, it may be envisaged to remove a fragment 8 of molecule 6 by removing one or more atoms and one or more bonds that are not included in the shortest paths that connect adjacent rotatable bonds (Fig. 10). This simplification may allow to ease the problem drastically: in some cases, small molecules can be reduced to a sequence of few (< 10) rotatable bonds. This simplification may be applied in conjunction with the removal of terminal hydrogens (again see Figures 8 to 10). It is important to underline that after the removal of rigid structures it is essential to fix the associated dihedral angle (not being rotatable in nature). To do this, a bond 9 may need to be inserted in the molecule (Fig. 11) and consequently a related special edge may need to be inserted in the associated graph representative of the modeled molecule every time a molecule fragment is replaced. This second simplification with molecule fragment removal as just described drastically reduces the difficulty of the docking problem. However, opting to have a lower number of vertices of the graph representing the molecule increases the possibility of having to discard some poses obtained from correlation of the binding molecule graph ($G_{mol}$) with the target molecule graph ($G_{grid}$), as explained below when discussing the weighted isomorphism between graphs.

In a variant of the fragment removal simplification (see Fig. 12), large rigid structures such as chains of aromatic compounds could be removed while maintaining only one edge connecting the two rotatable bonds to which these large rigid structures are connected. To increase the expressiveness of this simplification, the method may provide for adding a representative node 10 to the graph of the binding molecule, for example positioned in the center of mass. In this way, it is still possible to take into account the presence of a rigid structure in that position, without however having to maintain a large number of atoms (which lead to a high number of vertices and edges to be matched during the search for isomorphisms of the graph associated to the molecule).

After the above steps (or a part thereof depending on the case) have been completed the final binding molecule graph $G_{mol}$ is generated and the method described in this section ends (step 34 in Fig. 14)

D. From target molecule (e.g., protein or protein pocket) to target molecule graph (e.g., protein pocket graph)

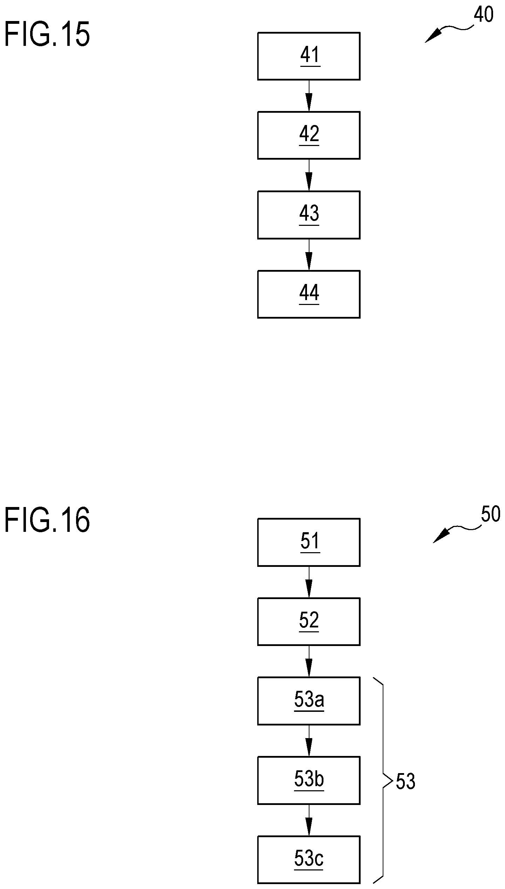

In accordance with a further aspect, a computer implemented method to model a target molecule is now described. A block diagram of the steps of this method is reported in Fig. 15, where the overall method is identified with reference numeral 40.

The description of the target molecule may already be available to the computing system 100 or for example it may obtained from a database 103 of molecules communicatively connected with the computing system 100. The computing system 100 may access database 103 containing a description of a plurality of target molecules in a given format (for example the database may contain descriptions of docking sites of molecules in .pbp file format) and then select the docking site or the target molecule to be modeled from the database (step 41 in Fig. 15).

Using techniques from the literature (e.g. CAVIAR, PASS, POCASA), descriptions of docking sites (a group of coordinates) can be found for a given target molecule, for example for a give protein. Docking sites are thus described as set of points and related coordinates. The computing system 100 starting from the coordinates of the set of points representative of the docking site, e.g., starting from a description of the docking site of a target molecule in .pbp file format, obtains a graph that represents the docking site geometry (step 42 in Fig. 15). In detail, the computer implemented method executed by computing system 100 provides for representing the docking site to be modeled as a graph including: a plurality of nodes, wherein each node is representative of a respective docking point of the docking site, and a plurality of edges, wherein each edge is representative of a respective connection between two docking points.

In summary, from the grid of points describing the pocket in the target molecule or protein, a graph ($G_{grid}$) may be generated, where each node of the graph is obtained from the obtained set of points. Regarding the connectivity (i.e., the arcs or edges) of the graph, different strategies can be adopted.

In accordance with an aspect, docking points $v$ are used as the vertices of a weighted graph associated to the protein (or other target molecule) pocket

$$G_{grid} = \{v, e_{u,v}, w_{u,v}\}$$

where $e_{u,v}$ are edges connecting points $u$ and $v$ with associated weights $w_{u,v}$.

In a currently preferred solution, the graph ($G_{grid}$) is a complete graph (i.e., fully connected), and the edge weights $w_{u,v}$ are defined as the Euclidean distance between docking points $u$ and $v$ as $w_{u,v} = d(u, v) = ||u - v||$, usually expressed in Angstrom or picometers. The distances may be obtained from the x, y, z coordinates of the docking points obtained from the molecule description, e.g., in .pbp file format as indicated above.

The constructed weighted graph ($G_{grid}$) hence identifies a 3D space-grid inside the pocket.

As mentioned the method requires the x, y, z position of the pocket points. For this reason, it is possible to use different file extensions, or simply a binary file with a list of points and their coordinates. The dataset that may be used for creating graphs of protein (protein pockets or other target molecule pockets) used consists of e.g., proteins stored in '.pdb' files. The Protein Data Bank file format allows to represent a database for three-dimensional structural data of (large) biological molecules, such as proteins. These protein datasets are usually accessible on the Internet via the website of organizations such as PDBe, PDBj, RCSB, and BMRB. In one of the implementations, the pocket points (and so the .pdb file) are generated via the Roll algorithm using the POCASA software.

In accordance with a further aspect the method may also comprise a pocket augmentation step (step 43 in Fig. 15). In fact, it could happen that between the edges of the pocket (grid) there are no edge weights $w_{u,v}$ that correspond to the length of the edges of the binding molecule. For this reason, it is possible to apply procedures to strategically insert additional points that compensate for the mentioned problem based on information of the binding molecule or ligand. The information related to the binding molecule, optionally to the ligand, to be hosted in the docking site of the target molecule comprises weights of edges of a graph representative of the binding molecule, optionally of the ligand. The augmentation step 43 comprises adding nodes in the graph representative of the docking site to allow in the graph ($G_{grid}$) representative of the docking site a distribution of the edge weights similar to that of the graph ($G_{mol}$) representative of the binding molecule, optionally of the ligand. For example if from the knowledge of the binding molecule or ligand it is determined that the pocket has edges that do not match with most or a significant portion of the edges of the binding molecule, it is possible to add intermediate points to the space grid of the binding molecule pocket and thus intermediate nodes to the graph representative of the target molecule docking site. In this example, a greater number of edges is obtained, potentially covering more distance values similar to edges of the graph representative of the binding molecule. In certain cases, it may also or alternatively be appropriate removing one or more nodes and one or more edges of the graph representative of the docking site based on information related to the binding molecule, optionally the ligand, to be hosted in the docking site of the target molecule (reduction step 44 in Fig. 15).

E. Optimal positional matching of a binding molecule in a docking site of a target molecule

A further aspect concerns a computer implemented method to find an optimal positional matching of a binding molecule (e.g., a ligand) in a docking site of a target molecule (e.g., a pocket of a protein). This method may be executed by computing system 100, which may be configured to perform the following steps (see Fig. 16 showing a block diagram of the method in question, wherein reference numeral 50 identifies the overall method):

- obtaining (step 51) a graph ($G_{mol}$) representative of the binding molecule, and
- obtaining (step 52) a graph ($G_{grid}$) representative of the docking site of the target molecule,

The graph ($G_{mol}$) representative of the binding molecule may be obtained from a molecule description using the computer implemented method (e.g., method 30 of Fig. 14) for modeling a binding molecule described in above section "*C. From binding molecule (e.g., ligand) to molecule graph*" of the present detailed.

On its turn, the graph ($G_{grid}$) representative of the docking site of the target molecule may be obtained from a target molecule description or docking site description using the computer implemented method (e.g., method 40 of Fig. 15) for modeling a docking site of a target molecule, for example of a target protein destined to receive a ligand, described above in section "D. *From target molecule (e.g., protein or protein pocket) to target molecule graph (e.g., protein pocket graph)*" of the present detailed description.

Using the binding molecule graph ($G_{mol}$) and the docking site or target molecule graph ($G_{grid}$) the method correlates the two graphs (step 53) for determining whether an optimal positional match exists between the binding molecule and the docking site of the target molecule by identifying one or more optimal three-dimensional poses of the binding molecule in the docking site. In accordance with a preferred solution, each three-dimensional pose defines conformation, position, and orientation of the binding molecule inside the docking site. In order to achieve this (namely to accomplish step 53), the method correlates the graph ($G_{grid}$) representative of the docking site of the target molecule with the graph ($G_{mol}$) representative of the binding molecule and determines whether an optimal positional match exists between the binding molecule and the docking site of the target molecule. More in detail, the problem of molecular docking, which is a complex optimization problem, is solved according to an aspect of the invention by mapping it to an isomorphism problem between graphs: in other words, the method here described correlates the two graphs ($G_{mol}$ and $G_{grid}$) and verifies if the graph of the binding molecule ($G_{mol}$) can be "incorporated" into the graph associated with the target molecule pocket ($G_{grid}$) identified as a possible docking site.

In greater detail the step 53 of correlating the graph ($G_{grid}$) representative of the docking site of the target molecule with the graph ($G_{mol}$) representative of the binding molecule comprises finding whether the graph ($G_{grid}$) representative of the docking site of the target molecule contains a subgraph ( $G_{grid}^{*}$ ) that is isomorphic to the graph ($G_{mol}$) representative of the binding molecule.

More specifically, according to a further aspect, correlating the graph ($G_{grid}$) representative of the docking site with graph ($G_{mol}$) representative of the binding molecule comprises finding whether the graph ($G_{grid}$) representative of the docking site of the target molecule contains a subgraph ( $G^*_{grid}$ ) that is a weighted isomorphism or close to a weighted isomorphism of the graph ($G_{mol}$) representative of the binding molecule. In accordance with another aspect, the step of finding whether the graph ($G_{grid}$) representative of the docking site of the target molecule contains a subgraph ( $G^*_{grid}$ ) that is a weighted isomorphism or close to a weighted isomorphism of the graph ($G_{mol}$) representative of the binding molecule identifies one or more optimal three-dimensional poses of the binding molecule in the docking site, by minimizing an error function correlated with the difference between the weights of the edges of the graph ($G_{mol}$) representing the binding molecule and the weights of the edges of the subgraph ( $G^*_{grid}$ ) of the graph ($G_{grid}$) representing the docking site. In other words, it may not be possible finding a 'perfect docking solution' and thus the method may accept a margin of error: thus an error function is defined and the error calculated and compared with a threshold of acceptability $\varepsilon$; depending upon the situation and the needs different error functions may be defined as long as they are based on the difference between the weights of the edges of the graph ($G_{mol}$) representing the binding molecule and the weights of the edges of the subgraph ( $G^*_{grid}$ ) of the graph ($G_{grid}$) representing the docking site.

In summary, according to a currently preferred aspect, the isomorphism necessary to solve the docking task is of the weighted type. The notion of isomorphism may thus be extended to the variant in which the graph must maintain not only the topology, but also the labels associated with the edges.

Thus, given the graph associated with the binding molecule:

$$\mathcal{G}_{mol} = (V_{mol}, E_{mol}, W_{mol})$$

and the graph associated to the docking site or space grid of the target molecule:

$$\mathcal{G}_{grid} = \left(V_{grid}, E_{grid}, W_{grid}\right)$$

the docking problem is considered solved when an optimal match between $G_{mol}$ and a sub-graph of the grid

$$\mathcal{G}^*_{grid} = \left(V^*_{grid}, E^*_{grid}\right) \mid V^*_{grid} \subseteq V_{grid}, E^*_{grid} \subseteq E_{grid} \cap \left(V^*_{grid} \times V^*_{grid}\right) \qquad (1)$$

is found, and therefore

$$\mathcal{G}^*_{grid} \simeq \mathcal{G}_{mol}.$$

Since the isomorphism is weighted, it should ideally hold that:

$$\sum_{(u,v)\in E_{mol}}\left(w_{u,\,v} - w_{u',v'}\right)^2 = 0 \qquad (2)$$

$$u' = f(u), u' \in V^*_{grid}$$

$$v' = f(v), v' \in V^*_{grid}$$

$$(u', v') \in E^*_{grid}$$

$$(u, v) \in \mathrm{E}_{mol}$$

This means that the weight associated to the edge $(u, v) \in E_{mol}$ must be equal to the weight of the edge $(u', v') \in E^*_{grid}$, where $u' = f(u)$ and $v' = f(v)$, and exists $(u', v') \in E^*_{grid} \subseteq E_{grid}$ for each $(u, v) \in E_{mol}$, for a given isomorphism $f$.

The terms in the above expressions are as follows:

- $G_{mol} = (V_{mol}, E_{mol}, W_{mol})$ is the graph representative of the binding molecule,
- $G_{grid} = (V_{grid}, E_{grid}, W_{grid})$ is the graph representative of the docking site,
- $G^*_{grid}$ is a subgraph of $G_{grid}$ and is an isomorphism of $G_{mol}$ on $G_{grid}$,
- $V_{mol}$ are the set of nodes of $G_{mol}$,
- $E_{mol}$ are the set of edges of $G_{mol}$,
- $W_{mol}$ are the set of weights of the of the edges of $G_{mol}$,
- $V_{grid}$ are the set of nodes of $G_{grid}$,
- $E_{grid}$ are the set of edges of $G_{grid}$,
- $W_{grid}$ are the set of weights of the edges of $G_{grid}$,
- $V^*_{grid}$ are the set of nodes of $G^*_{grid}$,
- $E^*_{grid}$ are the set of edges of $G^*_{grid}$,
- $W^*_{grid}$ are the set of weights of the edges of $G^*_{grid}$,
- $u, v$ are the nodes of $G_{mol}$,
- $u', v'$ are the nodes of $G_{grid}$,

Since the grid is of finite size, the existence of a perfect solution to the weighted subgraph isomorphism is not guaranteed. In other words, finding an isomorphism that perfectly respects the condition indicated in Equation (2) is unlikely, since the target molecule (protein) pocket graph is a discretization of the pocket space and has a limited number of points.

For this reason, it is possible to reconsider the problem in a two-phase one:

1. The first phase 53a consists in the search for a perfect topological (non-weighted) sub-graph isomorphism.
2. Then, in a second phase 53b, select among the solutions found the solutions which minimize the error introduced by the mismatch of the mapping of the weights of each edge of the graph ($G_{mol}$) on the graph ($G_{grid}$).

In other words, the correlation the graph ($G_{grid}$) representative of the docking site with graph ($G_{mol}$) representative of the binding molecule comprises:

- in the first phase 53a finding whether the graph ($G_{grid}$) representative of the docking site of the target molecule contains a subgraph ($G^*_{grid}$) that is a non-weighted isomorphism of the graph ($G_{mol}$) representative of the binding molecule, thereby identifying pre-optimal three-dimensional poses of the binding molecule in the docking site,
- then, in the second phase 53b, selecting among the pre-optimal three-dimensional poses identified in the first phase optimal three-dimensional poses that minimize the above error function (such as equation (2) above or other equations useful for determining a minimum in the discrepancy between weights of the edges of the two concerned graphs) correlated with the difference between the weights of the edges of the graph ($G_{mol}$) representing the binding molecule and the weights of the edges of the subgraph ($G^*_{grid}$) of the graph ($G_{grid}$) representing the docking site.

In the first phase (53a), identifying the pre-optimal three-dimensional poses of the binding molecule in the docking site comprises verifying that:

$$G^*_{grid} = \left(V^*_{grid}, E^*_{grid}\right) \mid V^*_{grid} \subseteq V_{grid}, E^*_{grid} \subseteq E_{grid} \cap \left(V^*_{grid} \times V^*_{grid}\right) \qquad (1)$$

In other words the gemoetric isomorphism must be cheched.

Then, in the second phase (53b), the step of selecting, among the pre-optimal three-dimensional poses identified in the first phase, optimal three-dimensional poses comprises finding the three-dimentional poses that minimize or zero the following error function:

$$\sum_{(u,v)\in E_{mol}}\left(w_{u,v} - w_{u',v'}\right)^2 \leq \mathcal{E} \ or = 0 \qquad (2)$$

with:

$$u' = f(u), u' \in V^*_{grid}$$

$$v' = f(v), v' \in V^*_{grid}$$

$$(u', v') \in E^*_{grid}$$

$$(u, v) \in \mathrm{E}_{mol}$$

meaning that, for the error function to be zero or a given threshold of acceptability, the weight associated to the edge $(u, v) \in E_{mol}$ must be equal or almost equal to the weight of the edge $(u', v') \in E^*_{grid}$, where $u' = f(u)$ and $v' = f(v)$, and exists $(u', v') \in E^*_{grid} \subseteq E_{grid}$ for each $(u, v) \in E_{mol}$, for a given isomorphism $f$; in other words, in accordance with this second methodology onche the non-weighted isomorphism (first pase= selects a number of potentially good candidates, the second phase selects the best ones accepting an error that may be defined by the operator.

The terms in the above expressions are as follows (as above already indicated):

- $G_{mol} = (V_{mol}, E_{mol}, W_{mol})$ is the graph representative of the binding molecule,
- $G_{grid} = (V_{grid}, E_{grid}, W_{grid})$ is the graph representative of the docking site,
- $G^*_{grid}$ is a subgraph of $G_{grid}$ and is an isomorphism of $G_{mol}$ on $G_{grid}$,
- $V_{mol}$ are the set of nodes of $G_{mol}$,
- $E_{mol}$ are the set of edges of $G_{mol}$,
- $W_{mol}$ are the set of weights of the of the edges of $G_{mol}$,
- $V_{grid}$ are the set of nodes of $G_{grid}$,
- $E_{grid}$ are the set of edges of $G_{grid}$,
- $W_{grid}$ are the set of weights of the edges of $G_{grid}$,
- $V^*_{grid}$ are the set of nodes of $G^*_{grid}$,
- $E^*_{grid}$ are the set of edges of $G^*_{grid}$,
- $W^*_{grid}$ are the set of weights of the edges of $G^*_{grid}$,
- $u, v$ are the nodes of $G_{mol}$,
- $u', v'$ are the nodes of $G_{grid}$.

Different metrics to define the error may be used in phase 53b to rank among the samples (i.e. binding molecules passing the first phase 53a) satisfying the isomorphism constraint, such as for example:

- Total Pairwise Distance (TPD);
- Root Mean Squared Deviation (RMSD);
- Average Bond Distortion (ABD).

Each scoring function has properties that can be used in different contexts and of course the skilled person may identify further ways of ranking. For example, the RMSD is a measurable metric only if the crystal pose of the ligand in the pocket is known. Below the TPD and ABD method are described in detail.

The Total pairwise Distance is calculated as the sum of the cumulative error deriving from the mapping of each edge $(u, v) \in E_{mol}$ of the graph associated with the molecule $G_{mol}$ with edge $(u, v) \in E_{grid}$ of the graph associated with the protein pocket $G_{grid}$.

Defining $G'_{grid}$ as a sample solution of the problem that satisfies the hard constraint:

$$G'_{grid} = \left(V'_{grid}, E'_{grid}\right) \mid V'_{grid} \subseteq V_{grid}, E'_{grid} \subseteq E_{grid} \cap \left(V'_{grid} \times V'_{grid}\right)$$

The *Total Pairwise Distance* of a given sample solution

$$G'_{grid}$$

constraint is calculated as:

$$TPD\left(G'_{grid}\right) = \sum_{(u,v) \in E_{mol}} \left(w_{u,v} - w_{u',v'}\right)^2,$$

$$w_{u,v} \in W_{mol}, w_{u',v'} \in W_{grid},$$

$$u \mapsto u' = f(u), u' \in V'_{grid},$$

$$v \mapsto v' = f(v), v' \in V'_{grid},$$

$$(u, v) \mapsto (u', v') \in E'_{grid}$$

That is, the TPD of a solution is equal to the error introduced by mapping a molecule edge to grid edges with different weights. Better mappings are those that allow lower TPD values.

Another version of the TPD function is the same as the last equation by applying, instead of the square of the distance, the absolute value:

$$TPD_{abs}\left(G'_{grid}\right) = \sum_{(u,v) \in E_{mol}} \left|w_{u,v} - w_{u',v'}\right|$$

The total pairwise distance is used within the objective function to define the landscape of the solution space.

One of the limitations of the Total Pairwise Distance is that, for the same candidate binding molecule by applying different simplifications, this value changes drastically, being a range that cumulatively sums the distortion of each weight associated with a bond $w_{u,v} \in W_{mol}$ once mapped on the grid representing the pocket $w_{u',v'} \in W_{grid}$.

Furthermore, this value strongly depends on the number of edges of the graph associated with the binding molecule, making it difficult to compare the performance of different binding molecule-target molecule pairs, especially if they have a different size.

For this reason, the *Average Bond Distortion (ABD)* may be used, which measures how much on average each bond (including bonds added to represent bond angles and dihedral angle constraints in case of double and triple bonds) is distorted in a particular sample (candidate binding molecule) that satisfies the isomorphism hard constraint (i.e. the first phase).

As for the Total Pairwise Distance, $G'_{grid}$ identifies a sample solution of the problem that satisfies the hard constraint (i.e. a potential candidate binding molecule that passes the first phase). The Average Bond Distortion of a sample solution is measured as:

$$ABD\left(\mathcal{G}'_{grid}\right) = \frac{1}{|V_{mol}|}\sqrt{TPD\left(\mathcal{G}'_{grid}\right)}$$

$$ABD\left(\mathcal{G}'_{grid}\right) = \frac{1}{|V_{mol}|}\sqrt{\sum_{(u,v)\in E_{mol}} \left(w_{u,v} - w_{u',v'}\right)^2},$$

$$w_{u,v} \in W_{mol}, w_{u',v'} \in W_{grid},$$

$$u \mapsto u' = f(u), u' \in V'_{grid},$$

$$v \mapsto v' = f(v), v' \in V'_{grid},$$

$$(u, v) \mapsto (u', v') \in E'_{grid}$$

That is, the ABD of a solution is equal to the root-mean TPD. It measures the average squared distortion of the edges of the molecules when mapped to grid edges with different weights. Better mappings are those that allow lower ABD values.

As for the TPD, instead of using the square of the difference, another version of the ABD metric can be defined applying the absolute value:

$$ABD\left(\mathcal{G}'_{grid}\right) = \frac{1}{|V_{mol}|}\sqrt{TPD_{abs}\left(\mathcal{G}'_{grid}\right)}$$

$$ABD\left(\mathcal{G}'_{grid}\right) = \frac{1}{|V_{mol}|}\sqrt{\sum_{(u,v)\in E_{mol}} \left|w_{u,v} - w_{u',v'}\right|}$$

In conclusion, the described method allows to find if there exist an optimal positional matching between a binding molecule (e.g., a ligand) and a docking site of a target molecule (e.g., a pocket of a protein), by correlating two graphs and then finding an optimal matching (if present) or a substantially optimal matching. Note that it is not necessary to select strictly one binding molecule (e.g., one ligand) and one target molecule (e.g., one protein): nothing prevents creating a multiplicity of graphs of respective binding molecules and a graph of the docking site of the target molecule and then testing the matching of even a huge number of binding molecules with one or more docking sites (and vice versa) and then ranking the matches using the error function.

F. From mathematical model to QUBO formulation

According to a further aspect, the correlation of the graph ($G_{grid}$) representative of the docking site with the graph ($G_{mol}$) representative of the binding molecule in order to solve the docking problem comprises constructing (step 53c) a corresponding Quadratic Unconstrained Binary Optimization (QUBO) problem.

As described in above section E., after obtaining .mol2 files for the binding molecules and .pdb files for the target molecules (proteins), respective graphs ($G_{mol}$) for each binding molecule and ($G_{grid}$) for each target molecule docking site are calculated.

Binding molecule graphs ($G_{mol}$) obtained from the .mol2 file, may be simplified, by removing the terminal hydrogens and certain rigid fragments keeping only the atoms and the edges which constitute the shortest path that connects the rotatable bonds adjacent to the rigid fragment. Alternatively, it is possible to add a node in the position of the center of mass in place of the removed atoms of the fragment, thus maintaining greater expressiveness.

Target molecule graphs ($G_{grid}$) obtained from the .pdb files may be augmented by adding nodes in strategic positions once some data from the candidate binding molecule have been obtained. For example, knowing the distribution of the edge lengths of graph ($G_{mol}$) obtained from the bond lengths and bond angle replacements, it is possible to insert nodes in the grid graph ($G_{grid}$) that allow a similar distribution of the edge weights (i.e., lengths).

The goal is to then find a law $f$ capable of associating to each node of the molecule graph a node of the grid graph.

To do this, a weighted subgraph isomorphism is used. Therefore, a graph $G_{grid}^* = \left(V_{grid}^*, E_{grid}^*\right)$ that is a valid isomorphism of graph $G_{mol}$ on $G_{grid}$ must be extracted. As defined in equation (1) $G_{grid}^*$ is a graph which vertices are a subset of the grid graph's vertices set $V_{grid}$; edges are a subset of the grid graph's set of edges $E_{grid}$ (which include just edges which are part of $V_{grid}^*$).

Topological isomorphism (disregarding the weights) finds solutions which can exists on the grid, but that does not mean that equation (2) holds. Though, since finding optimal isomorphisms is not an easy task, those solutions are still taken into consideration.

Given the two graphs $G_{mol}$ and $G_{grid}$ a Quadratic Unconstrained Binary Optimization (QUBO) problem is formulated defining a set of binary variables:

$$x_{u,u'} = \begin{cases} 1 & \textit{if vertex } u \textit{ is mapped to vertex } u' \\ 0 & \textit{otherwise} \end{cases}$$

$$\forall u \in V_{mol}, \quad \forall u' \in V_{grid}$$

The QUBO formulation is therefore defined as a weighted sum of two terms:

- isomorphism term: a hard constraint which ensures that each solution is a valid topological isomorphism of $G_{mol}$ on $G_{grid}$ (without considering the edge weights $W_{mol}$ and $W_{grid}$. This constraint ensures that each node or vertex of $G_{mol}$ is associated with one and only one node or vertex of $G_{grid}$, and that each edge of the molecule $(u, v) \in E_{mol}$ is mapped into an edge that actually belongs to the grid $(u', v') \in E_{grid}$.
- Optimization term: a term that evaluates a penalty term if the weight associated to an edge belonging to the molecule $(u, v) \in E_{mol}$ is mapped to an edge $(u', v') \in E_{grid}$ whose weight is different, $\forall (u, v) \in E_{mol}$.

$$\mathcal{H}_{MD} = AH_{iso} + H_{opt} \qquad (3)$$

The $A$ coefficient is introduced as a hyper-parameter of the problem: the isomorphism term and the optimization term are parts of the problem's Hamiltonian $\mathcal{H}_{MD}$, though, for a solution, the first must be satisfied in order to be considered as such.

To achieve this, the first part of the expression of the Hamiltonian, $H_{iso}$ is scaled to a factor of $A$, increasing its priority (i.e., heavily penalizing solutions which do not satisfy it).

F1. isomorphism term of the QUBO formulation

The hard constraint of the problem must ensure that, if guaranteed, a topological isomorphism of graph $G_{mol}$ on graph $G_{grid}$ is found. This term, therefore, does not take into account any of the values of the edge weights of the graphs.

The isomorphism term must ensure two properties of the valid mappings:

- Each node or vertex of the graph $G_{mol}$ must be mapped to one and only one node vertex of the grid graph $G_{grid}$:

$$H_1 = \sum_u \left(1 - \sum_{u'} x_{u,u'}\right)^2, \qquad u \in V_{mol}, u' \in V_{grid} \quad (4)$$

- Each edge of the graph $G_{mol}$ must be mapped to edges that are part of the grid graph

$$\mathcal{G}_{grid}:$$

$$:$$

$$H_2 = \sum_{(u,v)\in E_{mol}} \sum_{(u',v')\notin E_{grid}} x_{u,u'} x_{v,v'} \qquad (5)$$

The isomorphism term consists of the sum of the above two components:

$$H_{iso} = H_1 + H_2 \qquad (6)$$

The value of $H_{iso}$ is minimized when both conditions are hold. In the case of fully-connected graphs associated to the protein pocket (or pocket of other target molecule), the second term is equal to zero:

$$E_{grid} = \{(u',v') \mid V_{grid} \times V_{grid}, \ u' \neq v'\} \Longrightarrow H_2 = 0 \qquad (7)$$

$$since \ \nexists \ (u',v') \notin E_{grid}$$

F2. Optimization term of the QUBO formulation

The optimization term objective is to evaluate solutions which are valid isomorphisms of graph

$$\mathcal{G}_{mol}$$

on graph $G_{grid}$. As this may be a weighted isomorphism, topological valid matching may not fulfill the optimality requirement of equation (2), i.e., that each edge weight $w_{u,v} \in W_{mol}$ is matched with an edge whose weight is identical $w_{u',v'} \in W_{grid}$.
For this reason, solutions which satisfy the hard constraint have an additional optimization score to be minimized.

$$H_{opt} = \sum_{u,v\in E_{mol}} \sum_{u',v'\in E_{grid}} \left(w_{u,v} - w_{u',v'}\right)^2 x_{u,u'} x_{v,v'} \qquad (8)$$

Which means that: if the binary variables $w_{u,u'}$ (which maps node $u \in V_{mol}$ on node $u' \in V_{grid}$) and $w_{v,v'}$ (which maps node $v \in V_{mol}$ on node $v' \in V_{grid}$) are equal to 1, then a score is evaluated equal to the squared module of the difference between the edge weights associated to the edges $(u, v) \in E_{mol} (w_{u,v} \in W_{mol})$ and the edges $(u', v') \in E_{grid} (w_{u',v'} \in W_{grid})$.
In other words, if an atom bond length or bond angle is distorted during the isomorphism, the value of $H_{opt}$ will be strictly greater than 0.

The complete Hamiltonian $\mathcal{H}_{MD}$ associated to the QUBO formulation of the molecular docking problem using weighted subgraph isomorphism is:

$$\mathcal{H}_{MD} = A H_{iso} + H_{opt}$$

$$= A(H_1 + H_2) + H_{opt}$$

$$= A\left(\sum_u \left(1 - \sum_{u'} x_{u,u'}\right)^2 + \sum_{(u,v)\in E_{mol}} \sum_{(u',v')\notin E_{grid}} x_{u,u'} x_{v,v'}\right)$$

$$+ \sum_{u,v\in E_{mol}} \sum_{u',v'\in E_{grid}} \left(w_{u,v} - w_{u',v'}\right)^2 x_{u,u'} x_{v,v'} \qquad (9)$$

Since $H_{iso}$ has to be considered an hard constraint, the hyper-parameter A is large enough to ensure $H_{iso}$ = 0.

Hyper-parameter A may be selected such that:

$$A \geq \max_{(u,v)\in E_{mol},(u',v')\in E_{grid}} \left(w_{u,v} - w_{u',v'}\right)^2$$

For example, $A$ may be the following:

$$A = 10 \cdot \text{max\_optimization\_coefficient}$$

$$\text{max\_optimization\_coefficient} = \max_{(u,v)\in E_{mol},(u',v')\in E_{grid}} \left(w_{u,v} - w_{u',v'}\right)^2$$

Other $A$ values may be selected by the skilled person depending upon the circumstances.

As already discussed a further aspect concerns computing system (100), comprising one or more processors; and a computer-readable storage device coupled to the one or more processors and having instructions stored thereon which, when executed by the one or more processors, cause the one or more processors to perform the methods described above and disclosed in the claims. Furthermore, an additional aspect concerns non-transitory computer-readable storage media couplable to one or more processors (e.g., the processors of system 100) and having instructions stored thereon which, when executed by the one or more processors, cause the one or more processors to perform the methods described herein and claimed in the appended claims.

While this specification contains many specifics, these should not be construed as limitations on the scope of the disclosure or of what may be claimed, but rather as descriptions of features specific to particular implementations. Certain features that are described in this specification in the context of separate implementations may also be implemented in combination in a single implementation. Conversely, various features that are described in the context of a single implementation may also be implemented in multiple implementations separately or in any suitable sub-combination. Moreover, although features may be described above as acting in certain combinations and even initially claimed as such, one or more features from a claimed combination may in some cases be excised from the combination, and the claimed combination may be directed to a sub-combination or variation of a sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems may generally be integrated together in a single software product or packaged into multiple software products.

A number of implementations have been described. Nevertheless, it will be understood that various modifications may be made without departing from the spirit and scope of the disclosure. For example, various forms of the flows shown above may be used, with steps re-ordered, added, or removed. Accordingly, other implementations are within the scope of the following claims.

**Claims**

1.   Computer implemented method for determining an optimal positional matching of a binding molecule in a docking site of a target molecule, optionally for determining an optimal positional matching of a ligand in a docking site of a target protein, the computer implemented method comprising the steps of:

- obtaining (51) a graph ($G_{mol}$) representative of the binding molecule,
- obtaining (52) a graph ($G_{grid}$) representative of the docking site of the target molecule,
- correlating (53) the graph ($G_{grid}$) representative of the docking site of the target molecule with the graph ($G_{mol}$) representative of the binding molecule for determining whether an optimal positional match exists between the binding molecule and the docking site of the target molecule.

2. Computer implemented method according to claim 1, wherein obtaining (51) the graph ($G_{mol}$) representative of the binding molecule comprises the steps of:

- receiving (31) a molecule description of the binding molecule to be modeled,
- representing (32) the binding molecule to be modeled as a graph ($G_{mol}$), wherein the graph ($G_{mol}$) representative of the binding molecule includes:

  • a plurality of nodes (1), wherein each node is representative of a respective atom of the binding molecule,
  • a plurality of first edges (2), wherein each first edge is representative of a bond between a respective pair of atoms.

3. Computer implemented method according to claim 2, wherein each first edge (2) has a weight representative of a length of the bond between the respective pair of atoms;
wherein the graph ($G_{mol}$) representative of the binding molecule further includes:

- one or more second edges (3), wherein each second edge is representative of a respective bond angle, wherein each bond angle is the angle between a respective pair of bonds adjacent to a same atom, and wherein each second edge (3) has a weight representative of an amplitude of the respective bond angle;
- one or more third edges (4), wherein each third edge is representative of a respective dihedral angle,

wherein each dihedral angle is formed between a plane defined by a first and a second bond (A-B; B-C) and a plane defined by said second bond and a third bond (B-C, C-D), wherein, in a sequence of consecutively connected first, second, third and fourth atoms (A, B, C, D), the first bond (A-B) is between the first atom and the seconds atom, the second bond (B-C) is a bond between the second atom and the third atom and the third bond is a bond between the third atom and the fourth atom with the first and second bonds adjacent to the second atom (B) and with the second and third bonds adjacent to the third atom (C).

4. Computer implemented method according to any one of claims 2 or 3, wherein the molecule description of the binding molecule to be modeled comprises at least the identification of:

- the atoms of the binding molecule,
- the bonds between the binding molecule atoms, and
- the type of said bonds;

wherein the step of receiving (31) a molecule description of the binding molecule to be modeled includes:

- accessing a database (103) containing a description of a plurality of molecules in a given format optionally wherein the database contains molecule descriptions in .mol2 file format;
- selecting the binding molecule to be modeled from the database; optionally selecting the binding molecule to be modeled from said database described in .mol2 file format.

5. Computer implemented method according to any one of claims 2-4, wherein before or during the step of representing (32) the binding molecule as a graph ($G_{mol}$), the method comprises a step of molecule simplification (33) of the binding molecule to be modeled comprising at least removing from the binding molecule to be modeled one or more atoms; wherein the step of molecule simplification (33) comprises one or more of the following:

- a sub-step of reducing the number of atoms of the binding molecule to be modeled, optionally by removing the terminal hydrogens,
- a sub-step of fragment removal comprising removal of one or more atoms and one or more bonds not included in a shortest path connecting adjacent rotatable bonds, and introducing one or more constraints to maintain any dihedral angle of the binding molecule to be modeled;
- a sub step of fragment replacement comprising removal of one or more atoms and one or more bonds not included in a shortest path connecting adjacent rotatable bonds and replacing the removed atoms and bonds with a virtual atom optionally placed at the center of mass of the removed atoms.

6. Computer implemented method according to any one of the preceding claims, wherein obtaining (52) the graph ($G_{grid}$) representative of the docking site of the target molecule comprises the steps of:

- receiving (41) a description of a docking site to be modeled,
- representing (42) the docking site to be modeled as a graph, wherein the graph includes:

• a plurality of nodes, wherein each node is representative of a respective docking point of the docking site,
• a plurality of edges, wherein each edge is representative of a respective connection between two docking points.

7. Computer implemented method according to claim 6, wherein each edge has a weight representative of the Euclidean distance between two connected docking points.

8. Computer implemented method according to any one of the preceding two claims, wherein the description of the docking site to be modeled comprises the spatial coordinates of a plurality of docking points which are part of the docking site; and
wherein receiving (41) the description of a docking site to be modeled comprises:

- accessing a database (103) containing a description of docking sites of a plurality of molecules in a given format optionally wherein the database contains descriptions of docking sites of molecules in .pbp file format;
- selecting the docking site to be modeled from the database; optionally selecting the docking site to be modeled from said database in .pbp file format.

9. Computer implemented method according to any one of the preceding three claims, wherein the method comprises an augmentation step (43) including:

- adding one or more nodes and one or more edges to said graph ($G_{grid}$) representative of the docking site based on information related to a binding molecule, optionally a ligand, to be hosted in the docking site of the target molecule; optionally wherein the augmentation step comprises adding nodes in the graph representative of the docking site to allow in the graph representative of the docking site a distribution of the edge weights similar to that of the graph representative of the binding molecule, optionally of the ligand.
and/or

wherein the method comprises a reduction step (44) including:

- removing one or more nodes and one or more edges to said graph ($G_{grid}$) representative of the docking site based on information related to a binding molecule, optionally a ligand, to be hosted in the docking site of the target molecule;

optionally wherein the reduction step includes, based on distribution of edge lengths of the graph representative of the binding molecule ($G_{mol}$), removing from the graph representative of the docking site ($G_{grid}$) edges which lengths do not match with, or differ more than a given tolerance limit from, any length of the edges of graph representative of the binding molecule ($G_{mol}$).

10. Computer implemented method according to any one of the preceding claims, wherein determining whether an optimal positional match exists between the binding molecule and the docking site of the target molecule comprises:

- identifying one or more optimal three-dimensional poses of the binding molecule in the docking site,

wherein each three-dimensional pose defines conformation, position, and orientation of the binding molecule inside the docking site.

11. Computer implemented method according to any one of the preceding claims, wherein correlating (53) the graph ($G_{grid}$) representative of the docking site with the graph ($G_{mol}$) representative of the binding molecule comprises:

- finding (53a) whether the graph ($G_{grid}$) representative of the docking site of the target molecule contains a subgraph ( $G^*_{grid}$ ) that is isomorphic to the graph ($G_{mol}$) representative of the binding molecule;
or

wherein correlating the graph ($G_{grid}$) representative of the docking site with graph ($G_{mol}$) representative of the binding molecule comprises:

- finding (53b) whether the graph ($G_{grid}$) representative of the docking site of the target molecule contains a subgraph ( $G^*_{grid}$ ) that is a weighted isomorphism or close to a weighted isomorphism of the graph ($G_{mol}$) representative of the binding molecule.

12. Computer implemented method according to claim 11, second alternative, wherein finding (53b) whether the graph ($G_{grid}$) representative of the docking site of the target molecule contains a subgraph ( $G^*_{grid}$ ) that is a weighted isomorphism or close to a weighted isomorphism of the graph ($G_{mol}$) representative of the binding molecule comprises:

- identifying one or more optimal three-dimensional poses of the binding molecule in the docking site, by minimizing an error function correlated with the difference between the weights of the edges of the graph ($G_{mol}$) representing the binding molecule and the weights of the edges of the subgraph ( $G^*_{grid}$ ) of the graph ($G_{grid}$) representing the docking site.

13. Computer implemented method according to any one of claims 10 to 12, wherein identifying one or more optimal three-dimensional poses of the binding molecule in the docking site comprises verifying:

$$\mathcal{G}^*_{grid} = \left(V^*_{grid}, E^*_{grid}\right) \mid V^*_{grid} \subseteq V_{grid}, E^*_{grid} \subseteq E_{grid} \cap \left(V^*_{grid} \times V^*_{grid}\right) \quad (1)$$

and minimizing or zeroing the following error function:

$$\sum_{(u,v) \in E_{mol}} \left(w_{u,v} - w_{u',v'}\right)^2 \leq \mathcal{E} \ or = 0 \quad\quad (2)$$

with:

$$u' = f(u), u' \in V^*_{grid}$$

$$v' = f(v), v' \in V^*_{grid}$$

$$(u', v') \in E^*_{grid}$$

$$(u, v) \in E_{mol}$$

meaning that for the error function to be zero, or below a given threshold of acceptability $\varepsilon$, the weight associated to the edge $(u, v) \in E_{mol}$ must be equal or almost equal to the weight of the edge $(u', v') \in E^*_{grid}$, where $u' = f(u)$

and $v' = f(v)$, and exists $(u', v') \in E^*_{grid} \subseteq E_{grid}$ for each $(u, v) \in E_{mol}$, for a given isomorphism $f$; wherein the terms in the above expressions are as follows:

- $G_{mol} = (V_{mol}, E_{mol}, W_{mol})$ is the graph representative of the binding molecule,
- $G_{grid} = (V_{grid}, E_{grid}, W_{grid})$ is the graph representative of the docking site,
- $G^*_{grid}$ is a subgraph of $G_{grid}$ and is an isomorphism of $G_{mol}$ on $G_{grid}$,
- $V_{mol}$ are the set of nodes of $G_{mol}$,
- $E_{mol}$ are the set of edges of $G_{mol}$,
- $W_{mol}$ are the set of weights of the of the edges of $G_{mol}$,
- $V_{grid}$ are the set of nodes of $G_{grid}$,
- $E_{grid}$ are the set of edges of $G_{grid}$,
- $W_{grid}$ are the set of weights of the edges of $G_{grid}$,
- $V^*_{grid}$ are the set of nodes of $G^*_{grid}$,
- $E^*_{grid}$ are the set of edges of $G^*_{grid}$,
- $W^*_{grid}$ are the set of weights of the edges of $G^*_{grid}$,
- $u, v$ are the nodes of $G_{mol}$,
- $u', v'$ are the nodes of $G_{grid}$.

**14.** Computer implemented method according to any one of the preceding claims from 10 to 12, wherein correlating (53) the graph ($G_{grid}$) representative of the docking site with the graph ($G_{mol}$) representative of the binding molecule comprises:

- in a first phase (53a) finding whether the graph ($G_{grid}$) representative of the docking site of the target molecule contains a subgraph $(G^*_{grid})$ that is a non-weighted isomorphism of the graph ($G_{mol}$) representative of the binding molecule, thereby identifying pre-optimal three-dimensional poses of the binding molecule in the docking site,
- then, in a second phase (53b), selecting among the pre-optimal three-dimensional poses identified in the first phase optimal three-dimensional poses that minimize an error function correlated with the difference between the weights of the edges of the graph ($G_{mol}$) representing the binding molecule and the weights of the edges of the subgraph $(G^*_{grid})$ of the graph ($G_{grid}$) representing the docking site.

**15.** Computer implemented method according to claim 14, wherein, in the first phase (53a), identifying the pre-optimal three-dimensional poses of the binding molecule in the docking site comprises verifying that:

$$\mathcal{G}^*_{grid} = \left(V^*_{grid}, E^*_{grid}\right) \mid V^*_{grid} \subseteq V_{grid}, E^*_{grid} \subseteq E_{grid} \cap \left(V^*_{grid} \times V^*_{grid}\right) \quad (1)$$

and, in the second phase (53b), selecting, among the pre-optimal three-dimensional poses identified in the first phase, optimal three-dimensional poses comprises finding the three-dimentional poses that minimize or zero the following error function:

$$\sum_{(u, v) \in E_{mol}} \left(w_{u, v} - w_{u', v'}\right)^2 \leq \varepsilon \; or = 0 \quad (2)$$

with:

$$u' = f(u), u' \in V^*_{grid}$$

$$v' = f(v), v' \in V^*_{grid}$$

$$(u', v') \in E^*_{grid}$$

$$(u, v) \in \mathrm{E}_{\mathrm{mol}}$$

meaning that, for the error function to be zero or a given threshold of acceptability, the weight associated to the edge $(u, v) \in E_{mol}$ must be equal or almost equal to the weight of the edge $(u', v') \in E^*_{grid}$, where $u' = f(u)$ and $v' = f(v)$, and exists $(u', v') \in E^*_{grid} \subseteq E_{grid}$ for each $(u, v) \in E_{mol}$, for a given isomorphism $f$;
wherein the terms in the above expressions are as follows:

- $G_{mol} = (V_{mol}, E_{mol}, W_{mol})$ is the graph representative of the binding molecule,
- $G_{grid} = (V_{grid}, E_{grid}, W_{grid})$ is the graph representative of the docking site,
- $G^*_{grid}$ is a subgraph of $G_{grid}$ and is an isomorphism of $G_{mol}G_{mol}$ on $G_{grid}$,
- $V_{mol}$ are the set of nodes of $G_{mol}$,
- $E_{mol}$ are the set of edges of $G_{mol}$,
- $W_{mol}$ are the set of weights of the of the edges of $G_{mol}$,
- $V_{grid}$ are the set of nodes of $G_{grid}$,
- $E_{grid}$ are the set of edges of $G_{grid}$,
- $W_{grid}$ are the set of weights of the edges of $G_{grid}$,
- $V^*_{grid}$ are the set of nodes of $G^*_{grid}$,
- $E^*_{grid}$ are the set of edges of $G^*_{grid}$,
- $W^*_{grid}$ are the set of weights of the edges of $G^*_{grid}$,
- $u, v$ are the nodes of $G_{mol}$,
- $u', v'$ are the nodes of $G_{grid}$.

16. Computer implemented method according to any one of the preceding claims, wherein correlating (53) the graph ($G_{grid}$) representative of the docking site with graph ($G_{mol}$) representative of the binding molecule comprises constructing (53c) a corresponding Quadratic Unconstrained Binary Optimization (QUBO) problem;
optionally wherein the Quadratic Unconstrained Binary Optimization (QUBO) problem is solved and/or the optimal three-dimensional poses are determined by a Quantum Annealing (QA) optimization process executed by a quantum annealer.

17. Computer implemented method according to claim 16 wherein, given the graph ($G_{grid}$) representative of the docking site and the graph ($G_{mol}$) representative of the binding molecule, constructing a corresponding Quadratic Unconstrained Binary Optimization (QUBO) problem comprises:

- defining a set of binary variables:

$$x_{u,u'} = \begin{cases} 1 & \text{if vertex } u \text{ is mapped to vertex } u' \\ 0 & \text{otherwise} \end{cases}$$

$$\forall u \in V_{mol}, \quad \forall u' \in V_{grid}$$

- mapping each node of the graph of the binding molecule ($G_{mol}$) to one and only one node of the docking site graph ($G_{grid}$):

$$H_1 = \sum_u \left(1 - \sum_{u'} x_{u,u'}\right)^2, \qquad u \in V_{mol}, u' \in V_{grid} \tag{4}$$

- mapping each edge of the graph of the binding molecule ($G_{mol}$) to edges of the docking site graph ($G_{grid}$):

$$H_2 = \sum_{(u,v) \in E_{mol}} \sum_{(u',v') \notin E_{grid}} x_{u,u'} x_{v,v'} \tag{5}$$

- defining an isomormism term:

$$H_{iso} = H_1 + H_2 \tag{6}$$

- defining an optimization term:

$$H_{opt} = \sum_{(u,v) \in E_{mol}} \sum_{(u',v') \in E_{grid}} \left(w_{u,v} - w_{u',v'}\right)^2 x_{u,u'} x_{v,v'} \tag{8}$$

meaning that if the binary variables

$x_{u,u'}$, which map node $u \in V_{mol}$ on node $u' \in V_{grid}$, and
$x_{v,v'}$, which maps node $v \in V_{mol}$ on node $v' \in V_{grid}$)

are equal to 1, then a score is evaluated equal to the squared module of the difference between the edge weights associated to the edges $(u, v) \in E_{mol}$ ($w_{u,v} \in W_{mol}$) and the edges $(u', v') \in E_{grid}$ ($w_{u',v'} \in W_{grid}$).

- defining a complete Hamiltonian $\left(\mathcal{H}_{MD}\right)$ associated to the Quadratic Unconstrained Binary Optimization (QUBO) problem formulation as:

$$\mathcal{H}_{MD} = A H_{iso} + H_{opt} = A(H_1 + H_2) + H_{opt} =$$

$$= A \left( \sum_u \left(1 - \sum_{u'} x_{u,u'}\right)^2 + \sum_{(u,v) \in E_{mol}} \sum_{(u',v') \notin E_{grid}} x_{u,u'} x_{v,v'} \right)$$

$$+ \sum_{u,v \in E_{mol}} \sum_{u',v' \in E_{grid}} \left(w_{u,v} - w_{u',v'}\right)^2 x_{u,u'} x_{v,v'} \tag{9}$$

with A being an optimization parameter.

18. Computer implemented method according to claim 17, wherein optimal three-dimensional poses that minimize an error function correlated with the difference between the weights of the edges of the graph ($G_{mol}$) representing the binding molecule and the weights of the edges of the subgraph $\left(G_{grid}^*\right)$ of the graph ($G_{grid}$) representing the docking site are found by minimizing said complete Hamiltonian $\left(\mathcal{H}_{MD}\right)$.

19. Computer implemented method for identifying from a list of numerous candidate binding molecules one or more binding molecules that represent a promising match in a docking site of a target molecule, optionally for identifying from a list of numerous ligands the one or more that represent promising matches with a protein pocket, the method comprising:

- executing the method according to any one of the preceding claims for each candidate binding molecule,
- identifying among the numerous candidate binding molecules one or more binding molecules that represent a promising match in the docking site of the target molecule as those binding molecules for which an optimal positional matching in the docking site of the target molecule has been determined.

20. A computing system, comprising:

one or more processors; and

a computer-readable storage device coupled to the one or more processors and having instructions stored thereon which, when executed by the one or more processors, cause the one or more processors to perform the method of any one of the preceding claims.

21. Non-transitory computer-readable storage media couplable to one or more processors and having instructions stored thereon which, when executed by the one or more processors, cause the one or more processors to perform the method of any one of claims 1 to 19.

FIG.1

FIG.2

FIG.3

FIG.4

Gmol

FIG.5

TORSION ANGLE

FIG.6

FIG.7

FIG.8

FIG.9

FIG.10

FIG.11

FIG.12

FIG.13

FIG.14

31

32a

32b

32c

32d

32e

33

34

32

30

FIG.15

41

42

43

44

40

FIG.16

51

52

53a

53b

53c

53

50

Europäisches
Patentamt

European
Patent Office

Office européen
des brevets

**EUROPEAN SEARCH REPORT**

Application Number

EP 23 21 0835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEONARDO BANCHI ET AL: "Molecular Docking with Gaussian Boson Sampling", ARXIV.ORG, CORNELL UNIVERSITY LIBRARY, 201 OLIN LIBRARY CORNELL UNIVERSITY ITHACA, NY 14853, 1 February 2019 (2019-02-01), XP081024328, * p. 1, Abstract p. 1, left column, last par. p. 1, right column, par. 1 p. 2, left column, par. 2 p. 2, right column, par. 2 p. 2, left column, par. 3 p. 2, Figure 1 and description thereof p. 3, Figure 2 and description thereof * | 1-21 | INV. G16B15/30 G16C20/50 |
| A | SOPHIA M N HÖNIG ET AL: "Small molecule superposition: A comprehensive overview on pose scoring of the latest methods", WILEY INTERDISCIPLINARY REVIEWS: COMPUTATIONAL MOLECULAR SCIENCE, JOHN WILEY & SONS, INC, HOBOKEN, USA, vol. 13, no. 2, 11 October 2022 (2022-10-11), page n/a, XP072610586, ISSN: 1759-0876, DOI: 10.1002/WCMS.1640 * the whole document * | 1-21 | |
| A | KEVIN MATO: "Quantum molecular unfolding", QUANTUM SCIENCE AND TECHNOLOGY, vol. 7, no. 3, 9 June 2022 (2022-06-09), page 035020, XP093153526, ISSN: 2058-9565, DOI: 10.1088/2058-9565/ac73af Retrieved from the Internet: URL:https://iopscience.iop.org/article/10.1088/2058-9565/ac73af/pdf> * the whole document * | 1-21 | |

TECHNICAL FIELDS SEARCHED (IPC)

G16B
G16C

-/--

The present search report has been drawn up for all claims

| Place of search | Date of completion of the search | Examiner |
|---|---|---|
| Munich | 23 April 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

## EUROPEAN SEARCH REPORT

Application Number

EP 23 21 0835

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARITZA HERNANDEZ ET AL: "A Novel Graph-based Approach for Determining Molecular Similarity", ARXIV.ORG, CORNELL UNIVERSITY LIBRARY, 201 OLIN LIBRARY CORNELL UNIVERSITY ITHACA, NY 14853, 25 January 2016 (2016-01-25), XP080680147, * the whole document * | 1-21 | |

TECHNICAL FIELDS
SEARCHED     (IPC)

The present search report has been drawn up for all claims

| Place of search | Date of completion of the search | Examiner |
|---|---|---|
| Munich | 23 April 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **W. KLYNE** ; **V. PRELOG**. *Experientia*, 1960, vol. 16, 521-523